(19) Europäisches Patentamt
European Patent Office
Office européen des brevets

(11) Veröffentlichungsnummer: **0 271 040**
**A2**

(12) # EUROPÄISCHE PATENTANMELDUNG

(21) Anmeldenummer: **87118046.9**

(22) Anmeldetag: **07.12.87**

(51) Int. Cl.[4]: **C07D 487/04**, A61K 31/415, //(C07D487/04,235:00,209:00)

(30) Priorität: **11.12.86 DE 3642315**

(43) Veröffentlichungstag der Anmeldung: **15.06.88 Patentblatt 88/24**

(84) Benannte Vertragsstaaten: **AT BE CH DE ES FR GB GR IT LI LU NL SE**

(71) Anmelder: **BOEHRINGER MANNHEIM GMBH**
**Sandhofer Strasse 116**
**D-6800 Mannheim 31(DE)**

(72) Erfinder: **Friebe, Walter-Gunar, Dr. rer. nat.**
**Sophienstrasse 8**
**D-6800 Mannheim 1(DE)**
Erfinder: **Mertens, Alfred, Dr. rer. nat.**
**Beethovenstrasse 20**
**D-6905 Schriesheim(DE)**
Erfinder: **Strein, Klaus, Prof.**
**Eichenstrasse 45**
**D-6944 Hemsbach(DE)**
Erfinder: **Böhm, Erwin, Dr. med.**
**Hinterer Rindweg 37**
**D-6802 Ladenburg(DE)**

(54) **Neue Pyrrolobenzimidazole, Verfahren zu ihrer Herstellung, sowie Arzneimittel.**

(57) Pyrrolobenzimidazole der Formel I

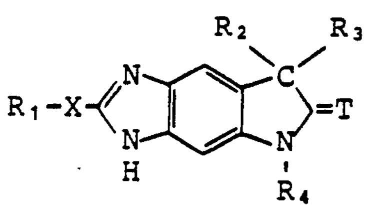

(I)

in welcher $R_1$ ein substituierter Phenylring, einen Naphthylrest oder einen gesaettigten oder ungesaettigten heterocyclischen Fuenfring mit 1-4 Heteroatomen oder einen gesaettigten oder ungesaettigten heterocyclischen Sechsring mit 1-5 Heteroatomen darstellt, wobei die Heteroatome gleich oder verscheiden sein koennen und Sauerstoff, Schwefel oder Stickstoff bedeuten und gewuenschtenfalls an einem oder mehreren Stickstoffatomen ein Sauerstoffatom tragen koennen, und die Fuenf-oder Sechsring gegebenenfalls durch eine oder mehrere Alkyl-, Alkoxy-, Alkylmercapto-, Hydroxy-, Nitro-, Amino-, Halogen-oder Cyangruppen substituiert oder mit einem Phenylring zu einem Bicyclus kondensiert sein koennen,
oder fuer den Fall, daß X eine Bindung darstellt, $R_1$ neben den oben genannten Gruppen auch ein Wasserstoffatom, eine Alkyl-, Cycloalkyl-, Alkenyl-, Cycloalkenyl-, Alkinyl-, Halogenalkyl-, Alkoxyalkyl-, Carboxyalkyl-, Alkoxycarbonylalkyl-, Aminoalkyl-, Hydroxyalkyl-, eine Hydroxy-, Mercapto-, Amino-, Alkylmercapto-, Alkylcarbonylamino-, Formylamino-, alkylsulfonylamino-, Formylaminoalkyl-, Alkoxycarbonylaminoalkyl-oder Alkylsulfonylaminoalkylgruppe bedeutet,
$R_2$ ein Wasserstoffatom, eine Alkyl-, Alkenyl-oder eine Cycloalkylgruppe bedeutet,

EP 0 271 040 A2

$R_3$ eine Alkyl-, Alkenyl-, oder Hydroxyalkylgruppe bedeutet oder mit $R_2$ zusammen eine Cycloalkylengruppe darstellt oder $R_2$ und $R_3$ zusammen eine Alkyliden-oder Cycloalkylidengruppe bilden,
$R_4$ ein Wasserstoffatom oder einen niederen Alkanoylrest darstellt,
X eine Bindung, eine Alkylen-, die Vinylen-, die Iminogruppe -NH-oder die Carbonylaminogruppe -CONH-bedeutet,
T fuer zwei Wasserstoffatome steht, wobei fuer den Fall, daß X die Iminogruppe -NH-oder die Carbonylaminogruppe -CONH-bedeutet, oder fuer den Fall, daß $R_1$ einen heterocyclischen Fuenf-oder Sechsring bedeutet, der mit einem Phenylring zu einem Bicyclus kondensiert ist, T auch ein Sauerstoffatom sein kann, deren Tautomere und deren physiologisch vertraegliche Salze anorganischer oder organischer Saeuren und Verfahren zur Behandlung von Herz-und Kreislauferkrankungen.

87118046.9

BOEHRINGER MANNHEIM GMBH

2882

Neue Pyrrolobenzimidazole, Verfahren zu ihrer Herstellung sowie Arzneimittel

Die vorliegende Erfindung betrifft neue Pyrrolobenzimidazole der allgemeinen Formel I,

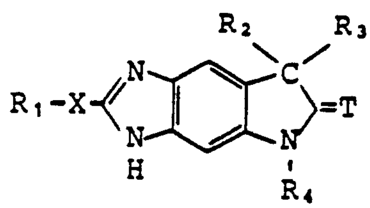

(I)

in welcher

$R_1$ einen Phenylring der allgemeinen Formel II darstellt,

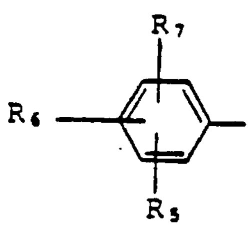

(II),

wobei $R_5$, $R_6$, $R_7$ gleich oder verschieden sein koennen und jeweils Wasserstoff, eine Alkansulfonyloxy-, Trifluormethansulfonyloxy-, Alkansulfonylamino-, Trifluormethansulfonylamino-, N-Alkyl-alkansulfonylamino-, N-Alkyltrifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, eine durch eine Amino-, Alkylamino-, Dialkylamino- oder cyclische Iminogruppe substituierte Sulfonylgruppe, wobei eine Methylengruppe in 4-Stellung durch ein Schwefel- oder Sauerstoffatom ersetzt sein kann, eine Alkylcarbonylamino-, Aminocarbonylamino- oder Alkylaminocarbonylaminogruppe, eine Alkylmercapto-, Alkylsulfinyl- oder Alkylsulfonylgruppe, eine Nitro-, Halogen-, Amino-, Hydroxy-, Alkyl-, Alkoxy-, Alkenyloxy-, Alkinyloxy-, Cyanalkyloxy-, Carboxyalkyloxy-, Alkoxycarbonylalkyloxy-, Dialkylamino-,

1-Imidazolyl-, Trifluormethyl- oder Cyangruppe sein koennen, oder $R_1$ einen Naphthylrest bedeutet
oder $R_1$ einen gesaettigten oder ungesaettigten heterocyclischen Fuenfring mit 1-4 Heteroatomen oder einen gesaettigten oder ungesaettigten heterocyclischen Sechsring mit 1-5 Heteroatomen darstellt, wobei die Heteroatome gleich oder verschieden sein koennen und Sauerstoff, Schwefel oder Stickstoff bedeuten und gewuenschtenfalls an einem oder mehreren Stickstoffatomen ein Sauerstoffatom tragen koennen, und die Fuenf- oder Sechsringe gegebenenfalls durch eine oder mehrere Alkyl-, Alkoxy-, Alkylmercapto-, Hydroxy-, Nitro-, Amino-, Halogen- oder Cyangruppen substituiert oder mit einem Phenylring zu einem Bicyclus kondensiert sein koennen,

oder fuer den Fall, daß X eine Bindung darstellt, $R_1$ neben den oben genannten Gruppen auch ein Wasserstoffatom, eine Alkyl-, Cycloalkyl-, Alkenyl-, Cycloalkenyl-, Alkinyl-, Halogenalkyl-, Alkoxyalkyl-, Carboxyalkyl-, Alkoxycarbonylalkyl-, Aminoalkyl-, Hydroxyalkyl-, eine Hydroxy-, Mercapto-, Amino-, Alkylmercapto-, Alkylcarbonylamino-, Formylamino-, Alkylsulfonylamino-, Formylaminoalkyl-, Alkoxycarbonylaminoalkyl- oder Alkylsulfonylaminoalkylgruppe bedeutet,

$R_2$ ein Wasserstoffatom, eine Alkyl-, Alkenyl- oder eine Cycloalkylgruppe bedeutet,

$R_3$ eine Alkyl-, Alkenyl-, oder Hydroxyalkylgruppe bedeutet oder mit $R_2$ zusammen eine Cycloalkylengruppe darstellt oder $R_2$ und $R_3$ zusammen eine Alkyliden- oder Cycloalkylidengruppe bilden,

$R_4$ ein Wasserstoffatom oder einen niederen Alkanoylrest darstellt,

X eine Bindung, eine Alkylen-, die Vinylen-, die Iminogruppe -NH- oder die Carbonylaminogruppe -CONH- bedeutet,

T fuer zwei Wasserstoffatome steht, wobei fuer den Fall, daß X die Iminogruppe -NH- oder die Carbonylaminogruppe

-CONH- bedeutet, oder fuer den Fall, daß $R_1$ einen heterocyclischen Fuenf- oder Sechsring bedeutet, der mit einem Phenylring zu einem Bicyclus kondensiert ist, T auch ein Sauerstoffatom sein kann, deren Tautomere und deren physiologisch vertraegliche Salze anorganischer oder organischer Saeuren und Verfahren zu ihrer Herstellung, sowie diese Verbindungen enthaltende Arzneimittel.

Da die Verbindungen der allgemeinen Formel I fuer den Fall, daß $R_2$ nicht gleich $R_3$ ist, ein asymmetrisches Kohlenstoffatom besitzen, sind auch Gegenstand der Erfindung die optisch aktiven Formen und racemischen Gemische dieser Verbindungen.

In den Anmeldungen EP-A 161 632, EP-A 186 010, EP-A 189 103 und DE-A 35 71 678 sind analoge Verbindungen beschrieben, in denen T ausschließlich Sauerstoff oder Schwefel bedeutet. Diese Verbindungen zeigen eine stark inotrope Wirkung. Die Verbindungen der vorliegenden Erfindung sind ebenfalls als Arzneimittel zur Behandlung von Herz- und Kreislauferkrankungen verwendbar, insbesondere beeinflussen sie die Thrombozyten- und Erythrozytenaggregation und/oder steigern die Herzkraft und/oder wirken vasodilatierend.

Bedeutet $R_1$ einen Phenylring der allgemeinen Formel II so kann der Alkylteil der bei $R_5$, $R_6$ und $R_7$ genannten Substituenten 1-5 Kohlenstoffatome enthalten, vorzugsweise 1-4 Kohlenstoffatome. Bevorzugt in diesem Sinne sind beispielsweise die Methansulfonyloxy-, Ethansulfonyloxy-, n-Propansulfonyloxy-, Isopropansulfonyloxy-, Trifluormethansulfonyloxy-, Methylsulfenylmethyl-, Ethylsulfenylmethyl-, n-Propylsulfenylmethyl-, Methylsulfinylmethyl-, Ethylsulfinylmethyl-, Methylsulfonylmethyl-, Ethylsulfonylmethyl-, n-Propylsulfonylmethyl-, Methansulfonylamino-, Ethansulfonylamino-, n-Propansulfonylamino-, Triflourmethansulfonylamino-, N-Methyl-methansulfonylamino-, N-Ethyl-methansulfonylamino-, N-Methyl-ethansulfonylamino-, N-Ethyl-ethansulfonylamino-, N-Isopropyl-ethansulfonylamino-, N-Methyl-n-propansulfonylamino-, N,n-Propyl-n-propansulfonylamino-, N-Methyl-trifluormethansulfonylamino-, N-Ethyl-trifluormethansulfonylamino-,

N-Isopropyl-trifluormethansulfonylamino-, Methoxycarbonyl-, Ethoxycarbonyl-, Propoxycarbonyl-, Isopropoxycarbonyl-, Methylaminocarbonyl-, Ethylaminocarbonyl-, Dimethylaminocarbonyl-, Di-n-propylaminocarbonyl-, N-Methyl-ethylaminocarbonyl-, Trifluormethyl-, Methylaminosulfonyl-, Ethylaminosulfonyl-, n-Propylaminosulfonyl-, n-Butylaminosulfonyl-, n-Pentylaminosulfonyl-, Dimethylaminosulfonyl-, Diethylaminosulfonyl-, Di-n-propylaminosulfonyl-, N-Methyl-isopropylaminosulfonyl-, Acetylamino-, Propionylamino-, Methylcarbonylamino-, Ethylaminocarbonylamino- oder Propylaminocarbonylaminogruppe, eine Methyl-, Ethyl-, Propyl-, Methoxy-, Ethoxy-, Propyloxy-, Allyloxy-, 2-Butenyloxy-, 3-Butenyloxy-, 2-Pentenyloxy-, Propargyloxy-, 2-Butinyloxy-, 3-Butinyloxy-, Cyanmethyloxy-, Cyanethyloxy-, Methoxycarbonylmethyloxy-, Methoxycarbonylethyloxy-, Methylmercapto-, Ethylmercapto-, Methylsulfinyl-, Ethylsulfinyl-, Methylsulfonyl- oder Ethylsulfonylgruppe.

Bei Sulfonylgruppen, die durch cyclische Iminogruppen substituiert sein koennen, sind bevorzugt die Morpholino-, Pyrrolidino-, Piperidino- und Hexamethyleniminosulfonylgruppen.
Insbesondere sind bevorzugt fuer

$R_1$ Wasserstoff, eine Alkylsulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyl-trifluormethylsulfonylaminogruppe, eine durch eine Hydroxy-, Alkoxy-, Amino-, Alkylamino oder Dialkylaminogruppe substituierte Carbonylgruppe oder eine durch eine Amino-, Dialkylamino- oder Morpholinogruppe substituierte Sulfonylgruppe, wobei jeder der vorstehend genannten Alkylteile 1 oder 2 Kohlenstoffatome enthalten kann, eine Nitro-, Cyan- oder Alkylaminosulfonylgruppe mit 1-4 Kohlenstoffatomen, eine Alkylcarbonylamino-, Aminocarbonylamino- oder N-Alkyl-aminocarbonylaminogruppe, eine Alkylmercapto-, Alkylsulfinyl- oder Alkylsulfonylgruppe, wobei jeder der vorgenannten Alkylteile 1 oder 2 Kohlenstoffatome ent-

halten kann, eine Halogen-, Amino-, Hydroxy-, Dialkylamino-, Alkyl-, Alkoxy-, Alkenyloxy- oder Alkinyloxygruppe vorzugsweise mit 1-3 Kohlenstoffatomen, eine Cyanmethyloxy- oder Methoxycarbonylmethyloxygruppe, die Trifluormethylgruppe oder die 1-Imidazolylgruppe,

fuer $R_6$ Wasserstoff, eine Alkylgruppe mit 1-3 Kohlenstoffatomen, eine Alkoxy- oder Dialkylaminogruppe mit 1 oder 2 Kohlenstoffatomen in jedem Alkylteil oder ein Halogenatom und

fuer $R_7$ Wasserstoff oder die Methoxygruppe.

Der Phenylteil kann 1 bis 3 der genannten Substituenten tragen.

Bevorzugte monosubstituierte Phenylverbindungen sind die Hydroxy-, $C_1$-$C_3$ Alkyl-, $C_1$-$C_3$ Alkoxy-, Allyloxy-, Propargyloxy-, Cyanmethyloxy-, Methoxycarbonylmethyloxy-, Halogen-, Nitro-, Cyan-, Aminocarbonyl-, Methoxycarbonyl-, Amino-, $C_1$-$C_3$ Dialkylamino-, $C_1$-$C_3$ Alkylmercapto-, $C_1$-$C_3$ Alkylsulfinyl-, $C_1$-$C_3$ Alkylsulfonyl-, $C_1$-$C_3$ Alkylsulfonyloxy- und die 1-Imidazolyl-phenyle, wobei der Substituent in 2-, 3- oder 4-Stellung stehen kann.

Bevorzugte disubstituierte Phenyle enthalten als Substituenten eine Alkansulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyl-trifluormethylsulfonylaminogruppe, eine durch eine Hydroxy-, Alkoxy-, Amino-, Alkylamino- oder Dialkylaminogruppe substituierte Carbonylgruppe oder eine durch eine Amino-, Dialkylamino- oder Morpholinogruppe substituierte Sulfonylgruppe, eine Alkylaminosulfonyl-, Alkylcarbonylamino-, Aminocarbonylamino- oder N-Alkyl-aminocarbonylaminogruppe, eine Hydroxy-, Alkyl-, Alkoxy-, Allyloxy-, Propargyloxy-, Cyanmethyloxy-,

Methoxycarbonylmethyloxy-, Cyan-, Halogen-, Nitro-, Amino-, Dialkylamino-, Alkylmercapto-, Alkylsulfinyl-, Alkylsulfonyl- oder eine 1-Imidazolylgruppe, wobei die beiden Substituenten gleich oder verschieden sein koennen und in 2,3-, 2,4-, 2,5-, 2,6-, 3,4- und 3,5-Stellung bevorzugt jedoch in 2,4-, 2,5- und 3,4-Stellung stehen koennen und die vorgenannten Alkylreste, allein oder in Kombination mit anderen Resten, 1-3 C-Atome aufweisen koennen.

Bevorzugter trisubstituierter Phenylrest ist der 3,4,5-Trimethoxyphenylrest.

Bedeutet $R_1$ einen heterocyclischen Fuenfring mit 1-4 Heteroatomen oder einen heterocyclischen Sechsring mit 1-5 Heteroatomen, wobei die Heteroatome der vorgenannten Fuenf- oder Sechsringe gleich oder verschieden sein koennen und Stickstoff, Sauerstoff oder Schwefel bedeuten und gegebenenfalls an einem oder mehreren Stickstoffatomen ein Sauerstoff tragen koennen, so sind in diesem Sinne bevorzugt der Pyrrol-, Furan-, Thiophen-, Pyrazol-, Imidazol-, Thiazol-, Isothiazol-, Oxazol-, Isoxazol-, Triazol-, Tetrazol-, Thiadiazol-, Oxadiazol-, Pyrazin-, N,N'-Dioxy-pyrazin-, Pyrimidin-, N,N'-Dioxy-pyrimidin-, Pyridazin-, Oxazin-, Thiazin-, Triazin-, Tetrazin-, Pyridin-, N-Oxy-pyridin-, Piperidin-, Piperazin-, Morpholin- und Thiomorpholinrest.

Alkyl-, Alkoxy- und Alkylmercapto-Substituenten in den heterocyclischen Fuenf- und Sechsring koennen 1-6, vorzugsweise 1-4 Kohlenstoffatome enthalten. Bevorzugt ist der Methyl-, Ethyl-, Methoxy-, Ethoxy-, Methylmercapto- und Ethylmercaptorest. Unter Halogen ist Fluro, Chlor und Brom, vorzugsweise Chlor zu verstehen.

Sind die heterocyclischen Fuenf- und Sechsringe mit einem Phenylring kondensiert, so sind bevorzugt der Indol-, Indazol-, Benzimidazol-, Chinolin-, Isochinolin-, Cinnolin-, Phthalazin-, Chinazolin-, Chinoxalin-, Benzofuran-, Benzothiophen-, Benzoxazol-, Benzisoxazol-, Benzothiazol- und der Benzisothiazolrest, außerdem der Naphthylrest.

Bedeutet X eine Bindung und $R_1$ einen Alkyl-, Alkenyl- oder Alkinylrest, so sind darunter gerade oder verzweigte Ketten mit bis zu zehn C-Atomen zu verstehen. Bevorzugt in diesem Sinne ist der Methyl-, Ethyl-, Propyl-, Butyl-, Pentyl-, Hexyl-, Vinyl-, Propenyl- und Propinylrest. Bedeutet X eine Bindung und $R_1$ einen Cycloalkyl- oder Cycloalkenylrest, so sind darunter Ringe mit drei bis sieben Gliedern zu verstehen. Bevorzugt in diesem Sinne ist der Cyclopropyl-, Cyclobutyl-, Cyclopentyl-, Cyclohexyl-, Cyclopentenyl- und Cyclohexenylrest. Bedeutet X eine Bindung und $R_1$ einen Halogenalkyl-, Alkoxyalkyl-, Carboxyalkyl-, Alkoxycarbonylalkyl-, Hydroxyalkyl-, Aminoalkyl-, Alkoxycarbonylaminoalkyl-, Alkylsulfonylaminoalkyl-, Alkylmercapto- oder einen Alkylcarbonylaminorest, so koennen die Alkyl- oder Alkoxygruppen 1 bis 6 C-Atomen enthalten. Unter Halogen ist Fluor und Chlor, vorzugsweise Fluor zu verstehen.

Bevorzugt in diesem Sinne ist der Trifluormethyl-, Ethoxymethyl-, Methoxyethyl-, Ethoxyethyl-, Carboxymethyl-, Carboxypropyl-, Carboxybutyl-, Methoxycarbonylmethyl-, Methoxycarbonylethyl-, Methoxycarbonylpropyl-, Ethoxycarbonylmethyl-, Ethoxycarbonylethyl-, Ethoxycarbonylpropyl-, Propoxycarbonylethyl-, Aminomethyl-, Aminoethyl-, Aminopropyl-, Aminobutyl-, Hydroxymethyl-, Hydroxyethyl-, Hydroxypropyl-, Hydroxybutyl-, Methylmercapto-, Ethylmercapto-, Propylmercapto-, Butylmercapto-, Acetylamino-, Propionylamino-, Butyloxycarbonylamino-, Methylsulfonylamino-, Formylaminopropyl-, Acetylaminopropyl-, Propionylaminopropyl- und der Methylsulfonylaminopropylrest.

Bedeuten in der allgemeinen Formel I $R_2$ und $R_3$ Alkylgruppen, so sind darunter gerade oder verzweigte Alkylketten mit 1-6 C-Atomen zu verstehen. Bevorzugt in diesem Sinne sind die Methyl-, Ethyl-, Propyl- und die Butylgruppe.

Bilden $R_2$ und $R_3$ zusammen mit dem C-Atom, an das sie gebunden sind einen carbocyclischen Ring, so sind darunter Ringe mit drei bis sieben Gliedern zu verstehen. Bevorzugt sind der Cyclopropan-, Cyclobutan-, Cyclopentan- und der Cyclohexanring.

Bedeutet in der allgemeinen Formel I $R_4$ einen Alkanoylrest, so sind darunter geradkettige, verzweigte und cyclische Alkanoylgruppen mit 1-7 C-Atomen zu verstehen.

Bevorzugt in diesem Sinne ist die Formyl-, Acetyl- und Propionyl-gruppe.

Bedeutet in der allgemeinen Formel I X eine Alkylengruppe, so sind darunter Alkylengruppen mit 1-4 C-Atomen zu verstehen. Bevorzugt in diesem Sinne ist die Methylen- und Ethylengruppe.

Besonders bevorzugte Verbindungen sind Verbindungen der allgemeinen Formel I

in der

$R_1$ den Phenylrest der allgemeinen Formel II bedeutet, in der $R_5$ ein Wasserstoffatom, die Methansulfonyloxy-, Trifluormethansulfonyloxy-, Methansulfonylamino-, Trifluormethansulfonylamino-, Methylmercapto-, Methylsulfinyl-, Methylsulfonyl-, Hydroxy-, Methyl-, Methoxy-, Propargyloxy-, Trifluormethyl- oder die 1-Imidazolylgruppe,

$R_6$ Wasserstoff, die Methyl-, Methoxy-, Dimethylamino- oder Chlorgruppe,

$R_7$ Wasserstoff oder die Methoxygruppe bedeutet oder

$R_1$ den Pyrrol-, Furan-, Thiophen-, Pyrazol-, Imidazol-, Isothiazol-, Thiazol-, Oxazol-, Triazol-, Tetrazol-, Thiadiazol-, Isoxazol-, Oxadiazol-, Pyridin-, N-Oxy-pyridin-, Pyrazin-, N,N'-Dioxypyrazin, Pyrimidin-, N,N'-Dioxypyrimidin-, Pyridazin-, Oxazin-, Thiazin-, Triazin-, Tetrazin-, Piperidin-, Piperazin-, Morpholin- oder Thiomorpholinrest darstellt sowie deren Methyl-, Ethyl-, Methoxy-, Ethoxy-, Methylmercapto-, Ethylmercapto- und Chlor-substitierten Derivate oder den Indol-, Indazol-, Chinolin- oder Isochinolinrest bedeutet oder einen Naphthylrest darstellt oder
$R_1$ fuer den Fall, daß X eine Bindung darstellt, neben den genannten Gruppen auch ein Wasserstoffatom, die Methyl-, Ethyl-, Propyl-, Butyl-,Pentyl-, Hexyl-, Propenyl-, Cyclopentyl-, Cyclohexyl-, Trifluormethyl-, Hydroxy-, Mercapto-, Methylmercapto-, Amino-, Acetamido- oder Formamidogruppe bedeutet,

$R_2$ und $R_3$ gleich sind und Methylgruppen bedeuten oder $R_2$ und $R_3$ zusammen mit dem Kohlenstoffatom, an das sie gebunden sind, einen Cyclopentanring oder Cyclohexanring bilden,

$R_4$ Wasserstoff, die Formyl-, Acetyl-, Propionyl- oder Butyrylgruppe darstellt und X eine Bindung, die Methylengruppe, die Iminogruppe oder die Carbonylaminogruppe bedeutet.

Das erfindungsgemaeße Verfahren zur Herstellung der Verbindungen der allgemeinen Formel I ist dadurch gekennzeichnet, daß man in an sich bekannter Weise entweder

a) eine Verbindung der allgemeinen Formel III

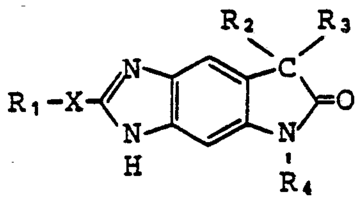 (III),

in welcher $R_1$, $R_2$, $R_3$, $R_4$ und X die obengenannte Bedeutung haben, reduziert oder

b) eine Verbindung der allgemeinen Formel IV a oder IV b

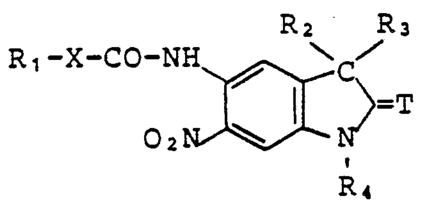 (IV a) 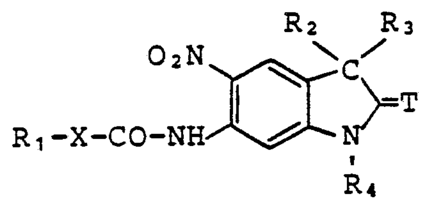 (IV b),

in welcher $R_1$, $R_2$, $R_3$, $R_4$, T und X die obengenannte Bedeutung haben, reduziert und cyclisiert oder

c) eine Verbindung der allgemeinen Formel V

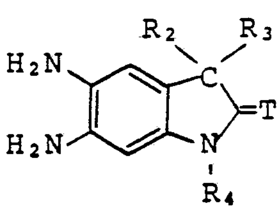 (V),

in welcher T, $R_2$, $R_3$ und $R_4$ die obengenannte Bedeutung haben, mit einer Verbindung der allgemeinen Formel VI

$$R_1'-X-\underset{\underset{W}{\|}}{C}-Y \qquad (VI),$$

in welcher X die oben angegebene Bedeutung besitzt, Y ein Wasserstoffatom, die Hydroxygruppe oder eine leicht abspaltbare Gruppe bedeutet, W fuer ein Sauerstoffatom steht oder gemeinsam mit Y ein Stickstoffatom darstellt und $R_1'$ die oben fuer $R_1$ angegebene Bedeutung besitzt mit der Ausnahme, daß fuer den Fall, daß X eine Bindung darstellt,

$R_1'$ nicht die Amino-, Hydroxy- oder Mercaptogruppe bedeutet, oder

mit Verbindungen umsetzt, die Carbonyl-, Thiocarbonyl- oder Iminogruppen uebertragen wie beispielsweise Phosgen, Thiophosgen, 1,1'-Carbonyldiimidazol, Chlorameisensaeureester, Kohlensaeureester, Harnstoff oder Bromcyan,

und anschließend gewuenschtenfalls

die erhaltene Verbindung der allgemeinen Formel I oder deren Tautomer in eine andere Verbindung der allgemeinen Formel I umwandelt und/oder die erhaltene Verbindung der allgemeinen Formel I in ein physiologisch vertraegliches Salz einer organischen oder anorganischen Saeure ueberfuehrt.

Die im Verfahren a genannte Reduktion wird vorzugsweise in einem aprotischen Loesungsmittel wie beispielsweise einem Ether wie Diethylether, Dimethoxyethan, Dioxan oder Tetrahydrofuran mit komplexen Metallhydriden wie beispielsweise Lithiumaluminiumhydrid bei Temperaturen zwischen 0 und 100°C, vorzugsweise bei der Siedetemperatur des Loesungsmittels, durchgefuehrt.

Die in Verfahren b genannte Reduktion wird vorzugsweise in einem Loesungsmittel oder Loesungsmittelgemisch wie Wasser, Methanol, Ethanol, Eisessig, Essigsaeureethylester oder Dimethylformamid mit Wasserstoff in Gegenwart eines Katalysators wie Raney-Nickel, Platin oder Palladium/Kohle, mit Metallen wie Eisen, Zinn oder Zink in Gegenwart einer Saeure, mit Salzen wie Eisen(II)sulfat, Zinn(II)chlorid, Natriumsulfid, Natriumhydrogensulfit oder Natriumdithionit oder mit Hydrazin in Gegenwart von Raney-Nickel bei Temperaturen zwischen 0 und 100°C, vorzugsweise jedoch bei Raumtemperatur, durchgefuehrt. Dabei entstehen meist unmittelbar die cyclisierten Verbindungen der allgemeinen Formel I.

Die Cyclisierung kann gewuenschtenfalls vervollstaendigt werden, indem man nach der Reduktion vorzugsweise in einem Loesungsmittel oder Loesungsmittelgemisch wie Ethanol, Isopropanol, Eisessig, Benzol, Toluol, Chlorbenzol, Glycol, Ethylenglycoldimethylether, Sulfolan oder Dimethylformamid auf Temperaturen zwischen 50 und 220°C, vorzugsweise jedoch auf die Siedetemperatur des Reaktionsgemisches, gegebenenfalls in Gegenwart eines Kondensationsmittels wie Phosphoroxychlorid, Thionylchlorid, p-Toluolsulfonsaeure, Salzsaeure, Schwefelsaeure, Phosphorsaeure, Polyphosphorsaeure oder gegebenenfalls auch in Gegenwart einer Base wie Natriumhydroxid, Natriumethylat oder Kalium-tert.-butylat erhitzt. Die Cyclisierung kann jedoch auch ohne Loesungsmittel und/oder Kondensationsmittel durchgefuehrt werden.

Unter den in Verfahren c genannten Verbindungen der allgemeinen Formel VI versteht man Aldehyde, Carbonsaeuren, Saeurehalogenide wie Saeurechloride, Carbonsaeureester wie Methyl- und Ethylester, Carbonsaeureamide und andere aktivierte Carbonsaeurederivate sowie Anhydride und Nitrile.
Ist die Verbindung der allgemeinen Formel VI ein Aldehyd, so findet die Umsetzung mit Verbindungen der allgemeinen Formel V unter oxidierenden Bedingungen statt, vorzugsweise in alkoholischen Medium unter Erwaermen zum Rueckfluß in Gegenwart von Luftsauerstoff und katalytischen Mengen Saeure, wie Toluolsulfonsaeure, oder in Gegenwart von Luftsauerstoff und eines Katalysators wie Braunstein in saurem Milieu wie z.B. in Eisessig bei Raumtempe-

ratur.

Ist die Verbindung der allgemeinen Formel VI eine Carbonsaeure oder ein Nitril, so findet die Umsetzung mit Verbindungen der allgemeinen Formel V in Gegenwart eines wasserentziehenden Mittels, vorzugsweise in Polyphosphorsaeure bei Temperaturen zwischen 50 und 250°C, vorzugsweise zwischen 100 und 200°C statt.

Ist die Verbindung der allgemeinen Formel VI ein Carbonsaeurederivat, so findet die Umsetzung mit Verbindungen der allgemeinen Formel V in einem inerten Loesungsmittel, vorzugsweise in Methylenchlorid oder Pyridin statt. Zur Vervollstaendigung der Cyclisierung erhitzt man anschließend in einem Loesungsmittel oder Loesungsmittelgemisch wie Ethanol, Isopropanol, Eisessig, Benzol, Chlorbenzol, Glycol, Diethylenglycoldimethylether, Sulfolan oder Dimethylformamid auf Temperaturen zwischen 50 und 250°C, vorzugsweise jedoch auf die Siedetemperatur des Loesungsmittels oder Loesungsmittelgemisches, gegebenenfalls in Gegenwart einen Kondensationsmittels wie Phosphoroxychlorid, Thionylchlorid, p-Toluolsulfonsaeure, Salzsaeure, Schwefelsaeure, Phosphorsaeure, Polyphosphorsaeure oder gegebenenfalls auch in Gegenwart einer Base wie Natriumhydroxid, Kaliummethylat oder Kalium-tert.-butylat. Die Cyclisierung kann jedoch auch ohne Loesungsmittel und/oder Kondensationsmittel durchgefuehrt werden.

Die in Verfahren c beschriebenen Umsetzungen mit Verbindungen, die Carbonyl-, Thiocarbonyl- oder Iminogruppen uebertragen, fuehrt man vorzugsweise so aus, daß man in eine salzsaure Loesung der Verbindungen der allgemeinen Formel V Phosgen einleitet oder Thiophosgen zugibt und bei Raumtemperatur stehen laeßt, oder die Verbindungen der allgemeinen Formel V mit Bromcyan oder Harnstoff ohne Loesungsmittel erhitzt, oder die Verbindungen der allgemeinen Formel V mit 1,1'-Carbonyldiimidazol in einem inerten Loesungsmittel wie Dioxan zum Sieden erhitzt.

Die als Ausgangsmaterial benoetigten Verbindungen der allgemeinen Formel III sind aus (int.Nr. 2653) bekannt oder koennen nach analogen Verfahren erhalten werden.

Die Verbindungen der allgemeinen Formeln IV a oder IV b lassen sich beispielsweise durch Umsetzung von Verbindungen der allgemeinen Formel VII a oder VII b

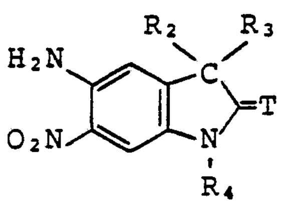 (VII a) 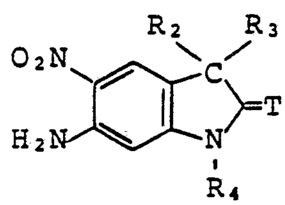 (VII b),

in der T, $R_2$, $R_3$ und $R_4$ die oben angegebene Bedeutung haben, mit Verbindungen der allgemeinen Formel VIII

$R_1$'-X-CO-Z (VIII),

in der $R_1$' und X die oben angegebene Bedeutung haben und Z eine Hydroxygruppe oder einen leicht abspaltbaren Rest darstellt, erhalten.

Die Verbindungen der allgemeinen Formel V werden durch Reduktion von Verbindungen der allgemeinen Formel VII a oder VII b erhalten.

Verbindungen der allgemeinen Formel VII a erhaelt man beispielsweise durch Nitrierung von 1-Acyl-5-acyl-amino-indolinderivaten nach literaturbekannten Methoden und anschließende Verseifung der 5-Acylgruppe.

Verbindungen der allgemeinen Formel VII b erhaelt man beispielsweise durch Nitrierung von 1-Acyl-6-acylamino-indolinderivaten nach literaturbekannten Methoden und anschließende Verseifung der 6-Acylgruppe.

Die Umwandlung von Verbindungen der allgmeinen Formel I zu anderen Verbindungen der allgemeinen Formel I trifft zum Beispiel zu

a) fuer die Umsetzung einer Verbindung der allgemeinen Formel I, in der $R_1$ eine Amino-, Aminoalkyl- oder cyclische Iminogruppe darstellt, oder einen mit einer Aminogruppe substituierten heterocyclischen Fuenf- oder Sechsring darstellt, oder einen Phenylring der allgemeinen Formel II darstellt, in der ein oder mehrere der Substituenten $R_5$, $R_6$, $R_7$ eine Aminogruppe darstellen, und/oder in der $R_4$ ein Wasserstoffatom

darstellt, mit Carbonsaeuren oder mit aktivierten Carbonsaeurederivaten wie Anhydriden oder Saeurehalogeniden zu Formylamino- oder Alkylcarbonylaminoderivaten. Umsetzungen mit Carbonsaeuren fuehrt man vorzugsweise in Gegenwart eines wasserentziehenden Mittels wie z.B. Polyphosphorsaeure oder eines mit Wasser ein azeotropes Gemisch bildenden Loesungsmittels wie Benzol oder Toluol durch. Umsetzungen mit aktivierten Carbonsaeurederivaten werden vorzugsweise in inerten Loesungsmitteln wie Methylenchlorid oder Pyridin bei Temperaturen zwischen 0°C und 250°C, vorzugsweise jedoch bei der Siedetemperatur des Loesungsmittels durchgefuehrt.

b) fuer die Umsetzung von Verbindungen der allgemeinen Formel I, in der $R_1$ eine Amino-, Aminoalkyl- oder cyclische Iminogruppe darstellt, oder $R_1$ einen mit einer Aminogruppe substituierten heterocyclischen Fuenf- oder Sechsring darstellt, wie er eingangs definiert ist, oder $R_1$ einen Phenylring der allgemeinen Formel II darstellt, in welcher einer der Substituenten $R_5$, $R_6$, $R_7$ eine Amino-, N-Alkylamino- oder Hydroxygruppe darstellt, mit einer Sulfonsaeure der allgemeinen Formel IX

$$R_8-SO_2OH \quad (IX),$$

in der $R_8$ eine Alkylgruppe mit 1-3 Kohlenstoffatomen oder eine Trifluormethylgruppe darstellt oder mit einem reaktionsfaehigen Derivat hiervon, zu Verbindungen der allgemeinen Formel I, in welcher die besagten Amino-, Aminoalkyl-, cyclischen Imino-, N-Alkylamino- oder Hydroxygruppen sulfoniert sind.

Die Umsetzung wird zweckmaeßigerweise in einem Loesungsmittel oder Loesungsmittelgemisch wie Methylenchlorid, Ether, Tetrahydrofuran, Dioxan oder Benzol gegebenenfalls in Gegenwart eines saeurebindenen Mittels wie Natriumcarbonat, Triethylamin oder Pyridin, wobei die beiden letzteren gleich-

zeitig auch als Loesungsmittel verwendet werden koennen, in Gegenwart eines die Saeure aktivierenden oder wasserentziehenden Mittels wie Thionylchlorid oder Phosphorpentachlorid, vorzugsweise jedoch mit einem reaktionsfaehigen Derivat einer Verbindung der allgemeinen Formel IX, z.B. mit deren Anhydrid oder Halogenid wie Methansulfonsaeurechlorid oder Ethansulfonsaeurechlorid, vorzugsweise bei Temperaturen zwischen 0 und 100°C, z.B. bei Temperaturen zwischen Raumtemperatur und 50°C, durchgefuehrt.

c) fuer die Umwandlung von Verbindungen der allgemeinen Formel I, in der $R_1$ eine Phenylgruppe der allgemeinen Formel II darstellt, wobei einer der Substituenten $R_5$, $R_6$, $R_7$ eine Alkylmercapto- oder Alkylsulfenylmethylgruppe mit 1-3 Kohlenstoffatomen im Alkylteil ist, zu Verbindungen der allgemeinen Formel I, in der $R_1$ eine Phenylgruppe und einer der Substituenten $R_5$, $R_6$, $R_7$ eine Alkylsulfinyl-, Alkylsulfonyl-, Alkylsulfinylmethyl- oder Alkylsulfonylmethylgruppe darstellt.

Diese Oxidation wird vorzugsweise in einem Loesungsmittel oder Loesungsmittelgemisch, z.B. in Wasser, Wasser/Pyridin, Aceton, Eisessig, verduennter Schwefelsaeure oder Trifluoressigsaeure, je nach dem verwendeten Oxidationsmittel zweckmaeßigerweise bei Temperaturen zwischen -80 und 100°C durchgefuehrt.

Zur Herstellung einer Alkylsulfinyl- oder Alkylsulfinylmethylverbindung der allgemeinen Formel I wird die Oxidation zweckmaeßigerweise mit einem Aequivalent des verwendeten Oxidationsmittel durchgefuehrt, z.B. mit Wasserstoffperoxid in Eisessig, Trifluoressigsaeure oder Ameisensaeure bei 0 bis 20°C oder in Aceton bei 0 bis 60°C, mit einer Persaeure wie Perameisensaeure in Eisessig oder Trifluoressigsaeure bei 0 bis 50°C oder mit m-Chlorperbenzoesaeure in Methylenchlorid oder Chloroform bei -20°C bis 60°C, mit Natriummetaperjodat in waessrigem Methanol oder Ethanol bei -15 bis 25°C, mit Brom in Eisessig oder waessriger

Essigsaeure, mit N-Brom-succinimid in Ethanol, mit tert.-Butylhypochlorit in Methanol bei -80°C bis -30°C, mit Jodbenzodichlorid in waessrigem Pyridin bei 0 bis 50°C, mit Salpetersaeure in Eisessig bei 0 bis 20°C, mit Chromsaeure in Eisessig oder in Aceton bei 0 bis 20°C und mit Sulfurylchlorid in Methylenchlorid bei -70°C, der hierbei erhaltene Thioether-Chlor-Komplex wird zweckmaeßigerweise mit waessrigem Ethanol hydrolysiert.

Zur Herstellung einer Alkylsulfonyl- oder Alkylsulfonylmethylverbindung der allgemeinen Formel I wird die Oxidation zweckmaeßigerweise mit einem bzw. mit zwei oder mehr Aequivalenten des verwendeten Oxidationsmittels durchgefuehrt, z.B. Wasserstoffperoxid in Eisessig, Trifluoressigsaeure oder in Ameisensaeure bei 20 bis 100°C oder in Aceton bei 0 bis 60°C, mit einer Persaeure wie Perameisensaeure, oder m-Chlorperbenzoesaeure in Eisessig, Trifluoressigsaeure, Methylenchlorid oder Chloroform bei Temperaturen zwischen 0 und 60°C, mit Salpetersaeure in Eisessig bei 0 bis 20°C, mit Chromsaeure oder Kaliumpermanganat in Eisessig, Wasser/Schwefelsaeure oder in Aceton bei 0 bis 20°C.

d) fuer die Umwandlung von Verbindungen der allgemeinen Formel I, in der $R_1$ eine Phenylgruppe der allgemeinen Formel II darstellt, wobei einer der Substituenten $R_5$, $R_6$, $R_7$ eine Carboxyl- oder Hydroxysulfonylgruppe darstellt, zu Verbindungen der allgemeinen Formel I, in der einer der Substituenten $R_5$, $R_6$ und $R_7$ eine durch eine Amino-, Alkylamino- oder Dialkylaminogruppe substituierte Carbonyl- oder Sulfonylgruppe darstellt. Dies geschieht durch Umsetzung mit einem Amin $HNR_9R_{10}$, wobei $R_9$ und $R_{10}$ gleich oder verschieden sein koennen und Wasserstoff oder $C_1$-$C_5$ Alkylgruppen bedeuten, oder mit einem reaktionsfaehigen Derivat hiervon. Vorteilhaft ist es, die Carboxylgruppe oder Hydroxysulfonylgruppe in ein reaktionsfaehiges Derivat, z.B. in einen Ester oder ein Saeurechlorid zu verwandeln und dann mit dem Amin $HNR_9R_{10}$ umzusetzen.

Die Umsetzung wird zweckmaeßigerweise in einem Loesungsmittel oder Loesungsmittelgemisch wie Methylenchlorid, Ethanol, Chloroform, Tetrachlorkohlenstoff, Ether, Tetrahydrofuran, Dioxan, Benzol, Toluol, Acetonitril oder Dimethylformamid, gegebenenfalls in Gegenwart eines die Saeure aktivierenden Mittels oder eines wasserentziehenden Mittels, z.B. in Gegenwart von Chlorameisensaeureethylester, Thionylchlorid, Phosphortrichlorid, Phosphorpentoxid, N'N-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiaeren organischen Base wie Triethylamin oder Pyridin, welche gleichzeitig als Loesungsmittel dienen koennen, bei Temperaturen zwischen -25 und 250°C, vorzugsweise jedoch bei Temperaturen zwischen -10°C und der Siedetemperatur des verwendeten Loesungsmittels durchgefuehrt werden, desweiteren kann waehrend der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

Besonders vorteilhaft wird jedoch die Umsetzung in einem entsprechenden Halogenid, z.B. dem Carbonsaeure- oder Sulfonsaeurechlorid, und einem entsprechenden Amin, wobei dieses gleichzeitig als Loesungsmittel dienen kann, und bei Temperaturen zwischen 0 und 50°C durchgefuehrt.

e) fuer die Umwandlung von Verbindungen der allgemeinen Formel I, in der $R_1$ einen Phenylring der allgemeinen Formel II darstellt und einer der Substituenten $R_5$, $R_6$ oder $R_7$ die Cyangruppe darstellt und/oder in der $R_4$ einen Alkanoylrest darstellt, zu Verbindungen der allgemeinen Formel I, in welcher $R_1$ einen Phenylring der allgemeinen Formel II darstellt und einer der Substituenten $R_5$, $R_6$ oder $R_7$ eine Alkoxycarbonylgruppe-, eine Aminocarbonylgruppe oder eine Carboxylgruppe bedeutet, und/oder in der $R_4$ ein Wasserstoffatom darstellt.

Diese Alkoholyse und/oder Hydrolyse wird entweder in Gegenwart einer Saeure wie Salzsaeure, Schwefelsaeure, Phosphorsaeure oder Trichloressigsaeure oder in Gegenwart einer Base wie Natriumhydroxid oder Kaliumhydroxid in einem geeigneten Loesungsmittel wie Wasser, Wasser/Methanol, Ethanol, Wasser/Ethanol, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, durchgefuehrt.

f) fuer die Alkylierung von Verbindungen der allgemeinen Formel I, in welcher $R_1$ einen Phenylring der allgemeinen Formel II darstellt, in der einer der Substituenten $R_5$, $R_6$ oder $R_7$ eine Hydroxy- oder Mercaptogruppe bedeutet, oder in welcher $R_1$ einen mit einer Hydroxy- oder Mercaptogruppe substituierten heterocyclischen Ring darstellt oder in der X eine Bindung darstellt und $R_1$ eine Hydroxy- oder Mercapto-

gruppe bedeutet. Dabei entstehen die entsprechenden Alkylmercapto- oder Alkyloxyverbindungen.

Die Reaktionen werden vorzugsweise in einem Loesungsmittel wie Aceton, Ether, Benzol, Toluol oder Dimethylformamid bei Temperaturen zwischen -30°C und +100°C, vorzugsweise bei Raumtemperatur in Gegenwart einer Base wie Kaliumcarbonat oder Natriumhydrid und eines Alkylierungsmittels wie Alkylhalogeniden oder Alkylsulfaten durchgefuehrt.

g) fuer die Reduktion von Verbindungen der allgemeinen Formel I, in welcher $R_1$ einen Pyridinring darstellt, zu Verbindungen der allgemeinen Formel I, in der $R_1$ den Piperidinring bedeutet. Diese Reduktionen fuehrt man vorzugsweise in alkoholischen Medium in Gegenwarte eines Katalysators wie Platin oder Palladium mittels Wasserstoff bei Normaldruck oder leicht erhoehtem Druck und einer Temperatur zwischen Raumtemperatur und 60°C.

h) fuer die Hydrierung einer Vinylverbindung ($X = -CH=CH-$) in eine entsprechende Ethylverbindung ($X = -CH_2-CH_2-$). Die Hydrierung wird vorzugsweise in einem Loesungsmittel wie Wasser, Wasser/Ethanol, Methanol, Eisessig, Essigsaeureethylester oder Dimethylformamid zweckmaeßigerweise mit Wasserstoff in Gegenwart eines Hydrierungskatalysators wie Raney-Nickel, Platin oder Palladium/Kohle durchgefuehrt.

i) fuer die Oxidation eines Fuenf- oder Sechsringes mit einem oder mehreren Stickstoffatomen zu den entsprechenden N-Oxiden. Die Oxidation wird zweckmaeßig mit einem oder mehreren Aequivalenten des verwendeten Oxidationsmittels durchgefuehrt, z.B. mit Wasserstoffperoxid in Eisessig, Trifluoressigsaeure oder in Ameisensaeure bei 20 - 100°C oder in Aceton bei 0 - 60°C, mit einer Persaeure wie Perameisensaeure oder m-Chlorperbenzoesaeure in Eisessig, Trifluoressigsaeure, Methylenchlorid oder Choroform bei Temperaturen zwischen 0 und 60°C.

Ferner koennen die erhaltenen Verbindungen der allgemeinen Formel I anschließend gewuenschtenfalls in ihre physiologisch vertraeglichen Saeureadditionssalze mit anorganischen oder organischen Saeuren ueberfuehrt werden. Als Saeuren kommen hierfuer beispielsweise Salzsaeure, Bromwasserstoffsaeure, Schwefelsaeure, Phosphorsaeure, Fumarsaeure, Bernsteinsaeure, Weinsaeure, Zitronensaeure, Milchsaeure, Maleinsaeure oder Methansulfonsaeure in Betracht.

Zur Herstellung von Arzneimitteln werden die Substanzen der allgemeinen Formel I in an sich bekannnter Weise mit geeigneten pharmazeutischen Traegersubstanzen, Aroma-, Geschmacks- und Farbstoffen gemischt und beispielsweise als Tabletten oder Dragees ausgeformt oder unter Zugabe entsprechender Hilfsstoffe in Wasser oder Oel, wie z.B. Olivenoel, suspendiert oder geloest.

Die erfindungsgemaeßen neuen Substanzen der allgemeinen Formel I und ihre Salze koennen in fluessiger oder fester Form enteral oder parenteral appliziert werden. Als Injektionsmedium kommt vorzugsweise Wasser zur Anwendung, welches die bei Injektionsloesungen ueblichen Zusaetze wie Stabilisierungsmittel, Loesungsvermittler oder Puffer enthaelt.

Derartige Zusaetze sind z.B. Tartrat- und Citratpuffer, Ethanol, Komplexbildner (wie Ethylendiamintetraessigsaeure und deren nicht toxische Salze) und hochmolekulare Polymere (wie fluessiges Polyethylenoxid) zur Viskositaetsregulierung. Feste Traegerstoffe sind z.B. Staerke, Lactose, Mannit, Methylcellulose, Talkum, hochdisperse Kieselsaeuren, hochmolekulare Fettsaeuren (wie Staerinsaeure), Gelatine, Agar-Agar, Calciumphosphat, Magnesiumstearat, tierische und pflanzliche Fette und feste hochmolekulare Polymere (wie Polyethylenglykole). Fuer orale Applikation geeignete Zubereitungen koennen gewuenschtenfalls Geschmacks- und Sueßstoffe enthalten.

Die erfindungsgemaeßen Verbindungen werden ueblicherweise in Mengen von 10-500 mg pro Tag bezogen auf 75 kg Koerpergewicht appliziert. Bevorzugt ist es, 2-3 mal pro Tag 1-2 Tabletten mit einem Wirkstoffgehalt von 5-200 mg zu verabreichen. Die Tabletten koennen auch retardiert sein, wodurch nur noch 1mal pro Tag 1-2 Tabletten mit 10-500 mg Wirkstoff gegeben werden muß. Der Wirkstoff kann auch durch Injektion 1-8mal pro Tag bzw. durch Dauerinfusion gegeben werden, wobei Mengen von 5-200 mg/Tag normalerweise ausreichen.

Bevorzugt im Sinne der Erfindung sind außer den in den Beispielen genannten Verbindungen die folgenden und deren Tautomere:

7,7-Dimethyl-2-(4-methyl-phenyl)-6,7-dihydro-3H,5H-pyrrolo-[2,3-f]benzimidazol

7,7-Dimethyl-2-(2-methoxy-4-methyl-phenyl)-6,7-dihydro-3H,5H-pyrrolo[2,3-f]benzimidazol

7,7-Dimethyl-2-(4-trifluormethyl-phenyl)-6,7-dihydro-3H,5H-pyrrolo[2,3-f]benzimidazol

7,7-Dimethyl-2-(2-methoxy-4-trifluormethyl-phenyl)-6,7-dihydro-3H,5H-pyrrolo[2,3-f]benzimidazol

7,7-Dimethyl-2-(4-methylthio-phenyl)-6,7-dihydro-3H,5H-pyrrolo[2,3-f]benzimidazol

7,7-Dimethyl-2-(2-methoxy-4-methylthio-phenyl)-6,7-dihydro-3H,5H-pyrrolo[2,3-f]benzimidazol

7,7-Dimethyl-2-(4-methylsulfinyl-phenyl)-6,7-dihydro-3H,5H-pyrrolo[2,3-f]benzimidazol

7,7-Dimethyl-2-(2-methoxy-4-methylsulfinyl-phenyl)-6,7-dihydro-3H,5H-pyrrolo[2,3-f]benzimidazol

7,7-Dimethyl-2-(4-methylsulfonyl-phenyl)-6,7-dihydro-3H,5H-pyrrolo[2,3-f]benzimidazol

7,7-Dimethyl-2-(4-methylsulfonyloxy-phenyl)-6,7-dihydro-3H,5H-pyrrolo[2,3-f]benzimidazol

7,7-Dimethyl-2-(2-methoxy-4-methylsulfonyloxy-phenyl)-6,7-dihydro-3H,5H-pyrrolo[2,3-f]benzimidazol

7,7-Dimethyl-2-(4-naphthyl)-6,7-dihydro-3H,5H-pyrrolo[2,3-f]-benzimidazol

7,7-Dimethyl-2-(2-methoxy-phenyl)-6,7-dihydro-3H,5H-pyrrolo[2,3-f]benzimidazol

7,7-Dimethyl-2-(1-imidazolyl)-6,7-dihydro-3H,5H-pyrrolo[2,3-f]-benzimidazol

7,7-Dimethyl-2-(4-thiomorpholinyl)-6,7-dihydro-3H,5H-pyrrolo[2,3-f]benzimidazol

Beispiel 1

2,7,7-Trimethyl-6,7-dihydro-3H,5H-pyrrolo[2,3-f]benzimidazol-dihydrochlorid

a) Eine Mischung aus 60.4 g (0.41 mol) 3,3-Dimethylindolin (CanJ.Chem. 29, 44 (1951)) und 90 ml Essigsaeureanhydrid wird 30 min zum Rueckfluß erhitzt. Man gießt auf Eis, filtriert und erhaelt 74.0 g (95 % d. Th.) N-Acetyl-3,3-dimethyl-indolin vom Schmp. 99-101°C.

b) Zu 130 ml konz. Schwefelsaeure gibt man bei 5°C 62.4 g (0.33 mol) N-Acetyl-3,3-dimethyl-indolin, tropft ein Gemisch aus 14.6 ml 100proz. Salpetersaeure und 14.6 ml konz. Schwefelsaeure unter Kuehlen zu, ruehrt 1 h im Eisbad, gießt auf Eis, filtriert und kristallisiert aus 2-Propanol um. Man erhaelt 68.8 g (89 % d. Th.) N-Acetyl-3,3-dimethyl-5-nitro-indolin vom Schmp. 172-174°C.

c) Eine Loesung von 50.0 g (0.21 mol) N-Acetyl-3,3-dimethyl-5-nitro-indolin in 500 ml Methanol und 500 ml Tetrahydrofuran wird ueber 5 ml Raney-Nickel bei 40°C und 1 bar Wasserstoffdruck hydriert. Nach Filtration, Einengen und Verreiben mit Ether verbleiben 36.3 g (83 % d. Th.) N-Acetyl-5-amino-3,3-dimethyl-indolin vom Schmp. 117-119°C.

d) Eine Mischung aus 44.0 g (0.22 mol) N-Acetyl-5-amino-3,3-dimethyl-indolin und 60 ml Essigsaeureanhydrid wird 30 min zum Rueckfluß erhitzt, abgekuehlt, auf Eis gegossen und filtriert. Den Filterrueckstand nimmt man in Methanol auf, versetzt bis zur basischen Reaktion mit Natriumbicarbonatloesung, filtriert, trocknet und engt ein. Man isoliert 48 g (91 % d. Th.) 5-Acetamido-1-acetyl-3,3-dimethyl-indolin vom Schmp. 190-191°C.

e) Zu 60 ml Essigsaeureanhydrid gibt man 24.6 g (0.1 mol) 5-Acetamido-1-acetyl-3,3-dimethyl-indolin und tropft unter Kuehlung 5.0 ml 100proz. Salpetersaeure zu. Man ruehrt 30 min bei 25-30°C nach, gießt auf Eis und filtriert. Man erhaelt 25.8 g (89 % d. Th.) 5-Acetamido-1-acetyl-3,3-dimethyl-6-nitro-indolin vom Schmp. 222-224°C.

f) 4.3 g 5-Acetamido-1-acetyl-3,3-dimethyl-6-nitro-indolin werden analog Beispiel 1 c) hydriert und ergeben 3.9 g (100 % d. Th.) 5-Acetamido-1-acetyl-6-amino-3,3-dimethyl-indolin vom Schmp. 88-90°C.

g) 3.9 g 5-Acetamido-1-acetyl-6-amino-3,3-dimethyl-indolin werden mit 100 ml gesaettigter ethanolischer Chlorwasserstoffloesung 3 h zum Rueckfluß erhitzt. Man engt ein und kristallisiert den Rueckstand aus Isopropanol um. Man erhaelt 3.4 g (83 % d. Th.) Titelverbindung vom Schmp. 255-260°C (Zers.).

## Beispiel 2

7,7-Dimethyl-1,2,6,7-tetrahydro-3H,5H-pyrrolo[2,3-f]-benzimidazol-2-on-hydrochlorid

a) Eine Mischung aus 37.5 g (0.13 mol) 5-Acetamido-1-acetyl-3,3-dimethyl-6-nitro-indolin (Bsp. 1 e), 250 ml Ethanol und 500 ml 2 N Natronlauge wird 6 h bei 50°C geruehrt. Nach Abfiltrieren verbleiben 29.6 g (92 % d. Th.) 1-Acetyl-5-amino-3,3-dimethyl-6-nitro-indolin vom Schmp. 220-221°C.

b) 15.0 g (0.06 mol) 1-Acetyl-5-amino-3,3-dimethyl-6-nitro-indolin werden in 300 ml Ethanol und 100 ml Tetrahydrofuran ueber 2 g Raney-Nickel bei 30°C und 1 bar Wasserstoffdruck hydriert. Nach Filtration und Einengen ver-

bleiben 12 g (91 % d. Th.) 1-Acetyl-5,6-diamino-3,3-dimethyl-indolin als Oel, aus denen mit ueberschuessiger etherischer Chlorwasserstoffloesung das Dihydrochlorid vom Schmp. 238-240°C erhaeltlich ist.

c) 6.0 g (0.02 mol) 1-Acetyl-5,6-diamino-3,3-dimethyl-indolin-dihydrochlorid werden in 50 ml 2 N Salzsaeure geloest. Durch diese Loesung leitet man waehrend 2 h Phosgen, spuelt anschließend mit Stickstoff, laeßt ueber Nacht stehen, filtriert ab und waescht den Niederschlag mit Wasser. Man isoliert 4.7 (96 % d. Th.) 5-Acetyl-7,7-dimethyl-1,2,6,7-tetrahydro-3H,5H-pyrrolo[2,3-f]-benzimidazol-2-on vom Schmp. oberhalb 300°C.

d) 3.0 g (13 mmol) 5-Acetyl-7,7-dimethyl-1,2,6,7-tetrahydro-3H,5H-pyrrolo[2,3-f]benzimidazol-2-on werden in 100 ml gesaettigter ethanolischer Chlorwasserstoffloesung 3 h zum Rueckfluß erhitzt. Man engt ein und kristallisiert den Rueckstand aus Ethanol um. Man erhaelt 2.3 g (74 % d. Th.) der Titelverbindung vom Schmp. oberhalb 300°C.

## Beispiel 3

### 7,7-Dimethyl-6,7-dihydro-2-phenyl-3H,5H-pyrrolo[2,3f]-benzimidazol-dihydrochlorid

a) Zu einer Loesung von 5.0 g (0.02 mol) 1-Acetyl-5-amino-3,3-dimethyl-6-nitro-indolin (Bsp. 2 a) in 130 ml Tetrahydrofuran und 7.0 ml Triethylamin tropft man 3.5 g (0.025 mol) Benzoylchlorid in 20 ml Tetrahydrofuran. Man erwaermt 6 h zum Rueckfluß, engt ein, versetzt mit Wasser, extrahiert mit Dichlormethan, engt den Extrakt ein und verreibt mit Ether. Man isoliert 6.4 g (91 % d. Th.) 1-Acetyl-5-benzamido-3,3-dimethyl-6-nitro-indolin vom Schmp. 186-187°C.

b) Durch Hydrierung der vorstehenden Verbindung analog Beispiel 2 b erhaelt man in quantitativer Ausbeute 1-Acetyl-6-amino-5-benzamido-3,3-dimethyl-indolin vom Schmp. 208-210°C.

c) Durch Behandlung mit ethanolischer Chlorwasserstoffloesung analog Beispiel 1 g erhaelt man aus der vorstehenden Verbindung in 97 % Ausbeute die Titelverbindung vom Schmelzpunkt 273-275°C.

Beispiel 4

5-Acetyl-7,7-dimethyl-6,7-dihydro-3H,5H-pyrrolo[2,3-f]-benzimidazol

Eine Mischung aus 3.0 g (0.01 mol) 1-Acetyl-5,6-diamino-3,3-dimethyl-indolin-dihydrochlorid (Bsp. 2 b) und 35 ml Formamid wird 40 min zum Rueckfluß erhitzt. Man engt ein, versetzt mit Wasser, extrahiert mit Dichlormethan und engt den Extrakt ein. Man erhaelt 2.6 g (85 % d. Th.) Titelverbindung vom Schmp. 214-216°C.

Beispiel 5

7,7-Dimethyl-6,7-dihydro-3H,5H-pyrrolo[2,3-f]benzimidazol-dihydrochlorid

Durch Behandlung mit ethanolischer Chlorwasserstoffloesung analog Beispiel 1 g erhaelt man aus der Verbindung des Beispiels 4 in 66 % Ausbeute die Titelverbindung vom Schmp. 248-250°C.

Beispiel 6

5-Acetyl-7,7-dimethyl-6,7-dihydro-2-trifluormethyl-3H,5H-pyrrolo[2,3-f]benzimidazol

Eine Mischung aus 60 g Polyphosphorsaeure 1.6 ml Trifluoressigsaeure, 18 g Phosphorpentoxid und 4.0 g (18 mmol) 1-Acetyl-5,6-diamino-3,3-dimethyl-indolin (Bsp. 2 b) wird 7 h bei 150-160°C geruehrt. Man laeßt auf 90°C abkuehlen, gießt auf Eis, laeßt ueber Nacht stehen und filtriert. Man erhaelt 1.7 g (31 %) der Titelverbindung vom Schmp. 290-291°C.

Beispiel 7

7,7-Dimethyl-6,7-dihydro-2-trifluormethyl-3H,5H-pyrrolo-[2,3-f]benzimidazol-dihydrochlorid

1.7 g (5.7 mmol) der Verbindung Bsp. 6 werden mit 40 ml 50proz. Trifluoressigsaeure 6 h zum Rueckfluß erhitzt. Man gießt auf Eis, waescht mit Dichlormethan, stellt ammoniakalisch, extrahiert mit Dichlormethan und engt den Extrakt ein. Man nimmt den Rueckstand in Aceton auf, versetzt mit ueberschuessiger etherischer Chlorwasserstoffloesung und filtriert 1.1 g (59 % d. Th.) Titelverbindung vom Schmp. 189-192°C ab.

Beispiel 8

7,7-Dimethyl-6,7-dihydro-2-ethyl-3H,5H-pyrrolo[2,3-f]-benzimidazol-dihydrochlorid

a) Eine Mischung aus 7.2 g (28 mmol) 1-Acetyl-5-amino-3,3-dimethyl-6-nitro-indolin (Bsp. 2 a) und 7 ml Propionsaeureanhydrid wird 30 min auf 120°C erhitzt. Man gießt

auf Eis und filtriert. Es verbleiben 8.1 g (95 % d. Th.) 1-Acetyl-3,3-dimethyl-6-nitro-5-propionamido-indolin vom Schmp. 178-180°C.

b) 7.5 g (25 mmol) der vorstehenden Verbindung werden analog Bsp. 1 c hydriert. Man erhaelt 6.1 g (89 % d. Th.) 1-Acetyl-6-amino-3,3-dimethyl-5-propionamido-indolin vom Schmp. 215-219°C.

c) 3.0 g (11 mmol) der vorstehenden Verbindung werden analog Beispiel 1 g mit ethanolischer Chlorwasserstoffloesung behandelt. Man isoliert 2.7 g (86 % d. Th.) Titelverbindung vom Schmp. 256-258°C.

Beispiel 9

7,7-Dimethyl-6,7-dihydro-2-(pyridin-3-yl)-3H,5H-pyrrolo-[2,3-f]benzimidazol

a) Eine Mischung aus 3.6 g (15 mmol) 1-Acetyl-5-amino-3,3-dimethyl-6-nitro-indolin (Bsp. 2 a), 150 ml Dichlormethan, 5.1 g Natriumbicarbonat und 5.1 g Nicotinoylchlorid-hydrochlorid wird 6 h zum Rueckfluß erwaermt, mit Wasser versetzt, mit Dichlormethan extrahiert und der Extrakt eingeengt. Nach Verreiben mit Ether erhaelt man 3.7 g (70 % d. Th.) 1-Acetyl-3,3-dimethyl-5-nicotinoylamino-6-nitro-indolin vom Schmp. 251-252°C.

b) Durch Hydrierung der vorstehenden Verbindung analog Beispiel 1 c erhaelt man mit 96 % Ausbeute 1-Acetyl-6-amino-3,3-dimethyl-5-nicotinoylamino-indolin als Oel.

c) 2.0g der vorstehenden Verbindung werden in 40 ml konz. Salzsaeure 3 h bei 90°C geruehrt. Man laeßt abkuehlen,

stellt ammoniakalisch und filtriert. Es verbleiben 0.8 g (52 % d. Th.) Titelverbindung vom Schmp. 213-215°C.

Beispiel 10

5-Acetyl-7,7-dimethyl-6,7-dihydro-2-(pyridazin-4-yl)-3H,5H-pyrrolo[2,3-f]benzimidazol

Zu 75 g Polyphosphorsaeure gibt man 3.1 g Pyridazin-4-carbonsaeure, 5.0 g (23 mmol) 1-Acetyl-5,6-diamino-3,3-dimethyl-indolin (Bsp. 2 b) und 22 g Phosphorpentoxid und erwaermt 7 h auf 150-160°C. Man gießt auf Eis, stellt ammoniakalisch, extrahiert mit Dichlormethan und engt den Extrakt ein. Es verbleiben 3.6 g (51 % d. Th.) Titelverbindung als dunkler Feststoff vom Schmp. 255-260°C.

Beispiel 11

7,7-Dimethyl-6,7-dihydro-2-(pyridazin-4-yl)-3H,5H-pyrrolo-[2,3-f]benzimidazol-dihydrochlorid

Durch Behandlung mit ethanolischer Chlorwasserstoffloesung analog Beispiel 1 g erhaelt man aus der Verbindung des Beispiels 10 in 45 % Ausbeute die Titelverbindung vom Schmp. 234-236°C.

## Beispiel 12

7,7-Dimethyl-6,7-dihydro-2-(pyridin-4-yl)-3H,5H-pyrrolo-[2,3-f]benzimidazol

Zu einer Suspension von 3.5 g Lithiumaluminiumhydrid in 100 ml Tetrahydrofuran tropft man bei Raumtemperatur eine Loesung von 5.3 g (19 mmol) 7,7-Dimethyl-6,7-dihydro-2-(pyridin-4-yl)-3H,5H-pyrrolo[2,3-f]benzimidazol-6-on. Man erwaermt 6 h zum Rueckfluß, zersetzt mit Kochsalzloesung, extrahiert mit Dichlormethan und Methanol, trocknet, engt ein und chromatographiert an Kieselgel. Man eluiert 1.9 g (38 % d. Th.) Titelverbindung vom Schmp. 147-149°C.

## Beispiel 13

In analoger Weise wie in Beispiel 12 beschrieben erhaelt man:

| | Bezeichnung | Ausbeute % | Schmp. °C (Loesungsm.) |
|---|---|---|---|
| a) | 7,7-Dimethyl-2-(3-methyl-pyrazol-5-yl)-6,7-dihydro-3H,5H-pyrrolo-[2,3-f]benzimidazol als Dihydrochlorid aus dem entsprechenden 6-Oxo-derivat | 76 | 243-47 (Methanol) |
| b) | 7,7-Dimethyl-2-(4-chinolinyl)-6,7-dihydro-3H,5H-pyrrolo[2,3-f]benzimidazol als Dihydrochlorid aus dem entsprechenden 6-Oxo-derivat (Bsp. 14 b) | 50 | 287-91 (Ethanol) |

Beispiel 14

In analoger Weise wie in Beispiel 10 beschrieben erhaelt man:

| | Bezeichnung | Ausbeute % | Schmp. °C (Loesungsm.) |
|---|---|---|---|
| a) | 2-(3-Chinolinyl)-7,7-dimethyl-6,7-dihydro-3H,5H-pyrrolo[2,3-f]benzimidazol-6-on aus 5,6-Diamino-3,3-dimethyl-indolin-2-on und Chinolin-3-carbonsaeure | 78 | ueber 300 (Methanol) |
| b) | 2-(4-Chinolinyl)-7,7-dimethyl-6,7-dihydro-3H,5H-pyrrolo[2,3-f]benzimidazol-6-on aus 5,6-Diamino-3,3-dimethyl-indolin-2-on und Chinolin-4-carbonsaeure | 67 | 210-213 (Ethanol) |
| c) | 7,7-Dimethyl-2-(2-indolyl)-6,7-dihydro-3H,5H-pyrrolo[2,3-f]benzimidazol-6-on aus 5,6-Diamino-3,3-dimethyl-indolin-2-on und Indol-2-carbonsaeure | 29 | ueber 350 (Ethanol) |
| d) | 7,7-Dimethyl-2-(3-pyridyl-amino)-6,7-dihydro-3H,5H-pyrrolo[2,3-f]benzimidazol-6-on aus 5,6-Diamino-3,3-dimethyl-indolin-2-on und 3-Cyanamino-pyridin | 22 | 206-208 (Methanol) |

Beispiel 14 (Fortsetzung)

| | Bezeichnung | Ausb. % | Schmelzpunkt °C (Lsg.mittel) |
|---|---|---|---|
| e) | 2'-(4-Chinolinyl)spiro(cyclopentan-1,7'-6',7'-dihydro-3'H,5'H-pyrrolo[2',3'-f]-benzimidazol)-6'-on aus 5',6'-Diamino-spiro(cyclopentan-1,3'-indolin)-2'-on und Chinolin-4-carbonsäure | 16 | 232-34 (Essigester) |
| f) | 5-Acetyl-7,7-dimethyl-6,7-dihydro-2-(imidazol-1-yl-methyl)-3H,5H-pyrrolo[2,3-f]-benzimidazol aus 1-Acetyl-5,6-diamino-3,3-dimethyl-indolin und Imidazol-1-essigsäure | 24 | über 280 (Dichlormethan) |
| g) | 7,7-Dimethyl-6,7-dihydro-2-(indol-3-yl-methyl)-3H,5H-pyrrolo[2,3-f]benzimidazol-6-on aus 5,6-Diamino-3,3-dimethyl-indolin-2-on und Indol-3-essigsäure | | |
| h) | 2'-(2-Indolyl)spiro(cyclopentan-1,7'-6',7'-dihydro-3'H,5'H-pyrrolo[2',3'-f]-benzimidazol)-6'-on aus 5',6'-Diamino-spiro(cyclopentan-1',3'-indolin)-2'-on und Indol-2-carbonsäure | | |
| i) | 2'-(4-Indolyl)spiro(cyclopentan-1,7'-6',7'-dihydro-3'H,5'H-pyrrolo[2',3'-f]-benzimidazol)-6'-on aus 5',6'-Diamino-spiro(cyclopentan-1',3'-indolin)-2'-on und Indol-4-carbonsäure | | |
| j) | 2'-(5-Indolyl)spiro(cyclopentan-1,7'-6',7'-dihydro-3'H,5'H-pyrrolo[2',3'-f]-benzimidazol)-6'-on aus 5',6'-Diamino-spiro(cyclopentan-1',3'-indolin)-2'-on und Indol-5-carbonsäure | | |

Beispiel 14 (Fortsetzung)

| Bezeichnung | | Ausb. % | Schmelzpunkt °C (Lsg.mittel) |
|---|---|---|---|
| k) | 2'-(6-Indolyl)spiro(cyclopentan-1,7'-6',7'-dihydro-3'H,5'H-pyrrolo[2',3'-f]-benzimidazol)-6'-on aus 5',6'-Diamino-spiro(cyclopentan-1',3'-indolin)-2'-on und Indol-6-carbonsäure | | |

Beispiel 15

7,7-Dimethyl-6,7-dihydro-2-(4-methoxybenzoylamino)-3H,5H-pyrrolo[2,3-f]benzimidazol-6-on

a) 20 g (104.6 mmol) 5,6-Diamino-3,3-dimethyl-indolin-2-on wurden in 300 ml Ethanol suspendiert, mit 12.2 g Bromcyan versetzt und 2.5 Std. bei 25°C gerührt. Der eingedampfte Rückstand wurde nochmals in Ethanol gelöst, das Ethanol weitgehend abdestilliert, mit Aceton versetzt und kristallisiert. Man erhält 25.7 g (82 %) 2-Amino-7,7-dimethyl-6,7-dihydro-3H,5H-pyrrolo[2,3-f]benzimidazol-6-on vom Schmp. 300-05°C als Hydrobromid.

b) 3 g (10.1 mmol) 2-Amino-7,7-dimethyl-6,7-dihydro-3H,5H-pyrrolo[2,3-f]benzimidazol-6-on x HBr wurden mit 2.5 g p-Anisoylchlorid in 50 ml Pyridin für 4 Std. bei 50°C gerührt. Anschließend wurde das Pyridin abdestilliert, der Rückstand mit Wasser durchgearbeitet, dekantiert, mit Ligroin durchgearbeitet und der Rückstand 2 x aus Ethanol umkristallisiert. Man erhält 1.4 g (40 %) der Titelverbindung mit einem Schmp. >300°C.

Beispiel 16

In analoger Weise wie in Beispiel 15 beschrieben erhält man:

| | Bezeichnung | Ausb. % | Schmelzpunkt °C (Lsg.mittel) |
|---|---|---|---|
| a) | 7,7-Dimethyl-6,7-dihydro-2-(benzoylamino)-3H,5H-pyrrolo-[2,3-f]benzimidazol-6-on | 34 | 315 (EtOH) |
| b) | 7,7-Dimethyl-6,7-dihydro-2-(2-methoxybenzoylamino)-3H,-5H-pyrrolo[2,3-f]benz-imidazol-6-on | 57 | 277-78 (EtOH) |
| c) | 7,7-Dimethyl-6,7-dihydro-2-(4-methylbenzoylamino)-3H,5H-pyrrolo[2,3-f]benzimidazol-6-on | 49 | >300 (EtOH) |
| d) | 7,7-Dimethyl-6,7-dihydro-2-(4-trifluormethylbenzoyl-amino)-3H,5H-pyrrolo[2,3-f]-benzimidazol-6-on | 57 | >300 (EtOH) |
| e) | 2'-Benzoylamino-spiro(cyclo-pentan-1,7'-6',7'-dihydro-3'H,5'H-pyrrolo[2',3'-f]-benzimidazol)-6'-on | 75 | >300 (Methanol) |
| f) | 2'-(4-Methoxybenzoylamino)-spiro(cyclopentan-1,7'-6',7'-dihydro-3'H,5'H-pyrrolo-[2',3'-f]benzimidazol)-6'-on | 62 | >300 (Methanol) |
| g) | 5-Acetyl-7,7-dimethyl-6,7-dihydro-2-(4-pyridyl-carbonylamino)-3H,5H-pyrrolo-[2,3-f]benzimidazol aus 5-Acetyl-2-amino-7,7-dimethyl-6,7-dihydro-3H,5H-pyrrolo[2,3-f]benzimidazol | 55 | über 280 (Wasser) |
| h) | 2'-(4-Pyridincarbonylamino)-spiro(cyclopentan-1,7'-6',7'-dihydro-3'H,5'H-pyrrolo-[2',3'-f]benzimidazo)-6'-on | 56 | >300 (Methanol) |

Beispiel 17

6,7-Dihydro-7,7-dimethyl-2-(4-methoxyphenylamino)-3H,5H-pyrrolo[2,3-f]benzimidazol-6-on

Zu einer Lösung von 3.8 g 5,6-Diamino-3,3-dimethyl-indolin-2-on in 20 ml trockenem Ethanol tropfte man 3 ml 4-Methoxyphenylisothiocyanat. Man rührte drei Stunden bei 60°C, entfernte das Lösungsmittel im Vakuum, suspendierte den Rückstand in Wasser und extrahierte mit Dichlormethan. Man trocknete die organische Phase über Natriumsulfat, filtrierte und entfernte das Lösungsmittel i. Vak.. Man erhielt 7.5 g Rückstand, von dem man 6.8 g in 1 L Dichlormethan löste, 8 ml Triethylamin und 16 g Quecksilber-II-chlorid zugab und 3 Tage unter Rückfluß zum Sieden erhitzte. Man entfernte das Lösungsmittel i. Vakuum, suspendierte den Rückstand in 100 ml Ethanol, gab 30 ml konz. Salzsäure zu und sättigte mit Schwefelwasserstoff. Man erhitzte 10 min zum Rückfluß, filtrierte heiß das ausgefallene Quecksilbersulfid ab und wusch mit heißem Ethanol nach. Das Filtrat engte man im Vakuum zur Trockene ein, löste in 100 ml Wasser, filtrierte ungelöste Bestandteile ab, stellte das Filtrat mit 2 N Ammoniak alkalisch, saugte die Base ab und wusch mit Wasser nach. Man reinigte über eine Kieselgelsäule (Laufmittel: 1,1,1-Trichlorethan/methanol. Ammoniak = 15:1), engte entsprechende Fraktionen i. Vakuum ein, nahm das Öl in heißem Ethanol auf, setzte bis zur beginnenden Trübung Ether zu und ließ kristallisieren. Man erhielt 2.8 g der Titelverbindung mit dem Schmp. 286-288°C.

Beispiel 18

In analoger Weise wie in Beispiel 17 beschrieben erhält man:

| Bezeichnung | | Ausb. % | Schmelzpunkt °C (Lsg.mittel) |
|---|---|---|---|
| a) | 6',7'-Dihydro-2'-phenyl-amino-spiro(cyclopentan-1,7'-3'H,5'H-pyrrolo[2',3'-f]benzimidazol)-6'-on aus 5',6'-Diamino-spiro[cyclo-pentan-1,3'-indolin]-2-on und Phenylisothiocyanat | 34 | 290-294 (Dichlormethan) |

## Beispiel 19

6,7-Dihydro-7,7-dimethyl-2-(3-indolyl)-3H,5H-pyrrolo-[2,3-f]benzimidazol-6-on

3.1 g 5,6-Diamino-3,3-dimethyl-indolin-2-on und 2.5 g Indol-3-aldehyd rührte man in 150 ml Methanol und 4 ml Eisessig vier Tage bei Raumtemperatur. Man entfernte das Lösngsmittel i. Vak. und reinigte den Rückstand säulenchromatographisch an Kieselgel. Die entsprechenden Fraktionen wurden vereinigt, das Lösungsmittel i. Vak. entfernt, der Rückstand (3.7 g gelbes Öl) in Methanol gelöst, bis zur beginnenden Trübung Dichlormethan zugesetzt und kristallisieren gelassen. Man erhielt 3.1 g der Titelverbindung mit dem Schmp. 243-245°C.

## Beispiel 20

5-Acetyl-7,7-dimethyl-2-(2-methoxy-4-methylsulfonyl-phenyl)-6,7-dihydro-3H,5H-pyrrolo[2,3-f]benzimidazol

a) Eine Mischung aus 6.6 g (30 mmol) 1-Acetyl-5,6-diamino-3,3-dimethyl-indolin, 150 ml Ethanol und 11.4 g (40 mmol) Natrium-α-Hydroxy-(2-methoxy-4-methylthio-phenyl)methansulfonat wird unter Einleiten von Luft 3 h bei Raumtemperatur gerührt. Man versetzt mit Wasser und Sodalösung, extrahiert mit Dichlormethan, trocknet und engt ein. Nach Chromatographie an Kieselgel erhält man 7.6 g (66 % d.Th.) 5-Acetyl-7,7-dimethyl-2-(2-methoxy-4-methylthio-phenyl)-6,7-dihydro-3H,5H-pyrrolo[2,3-f]benzimidazol vom Schmp. 105-107°C.

b) 3.8 g (10 mmol) der vorstehenden Verbindung werden in 70 ml Essigsäure und 7 ml 30proz. Wasserstoffperoxid 24 h bei Raumtemperatur gerührt. Man versetzt mit Wasser, engt im Vakuum ein und chromatographiert an Kieselgel. Man isoliert 2.3 g (56 % d.Th.) der Titelverbindung vom Schmp. 223-225°C.

Beispiel 21

5-Acetyl-7,7-dimethyl-2-phenyl-6,7-dihydro-3H,5H-pyrrolo[2,3-f]benzimidazol

Eine Mischung aus 2.2 g (10 mmol) 1-Acetyl-5,6-diamino-3,3-dimethyl-indolin, 200 ml Methanol, 5.2 ml Essigsäure, 1.0 ml Benzaldehyd und 50 mg Mangandioxid wird 3 h bei Raumtemperatur unter Einleiten von Luft gerührt. Man filtriert, engt ein und chromatographiert an Kieselgel. Es verbleiben nach Einengen der entsprechenden Fraktion und Anreiben mit Ether 0.8 g (26 % d.Th.) Titelverbindung vom Schmp. 225-227°C.

Beispiel 22

In analoger Weise wie in Beispiel 21 beschrieben erhält man:

| | Bezeichnung | Ausb. % | Schmelzpunkt °C (Lsg.mittel) |
|---|---|---|---|
| a) | 5-Acetyl-7,7-dimethyl-2-n-hexyl-6,7-dihydro-3H,5H-pyrrolo[2,3-f]benzimidazol aus 1-Acetyl-5,6-diamino-3,3-dimethyl-indolin und Heptanal | 35 | 188-190 (Dichlor-methan) |
| b) | 5'-Acetyl-2'-(4-methoxy-phenyl)-6',7'-dihydro-spiro-(cyclopentan-1,7'-3'H,5'H-pyrrolo[2',3'-f]benzimidazol) aus 1'-Acetyl-5',6'-diamino-spiro[cyclopentan-1,3'-indolin] und 4-Methoxy-benzaldehyd | 47 | über 280 (Ether) |
| c) | 5'-Acetyl-2'-(4-methoxy-phenylmethyl)-6',7'-dihydro-spiro(cyclopentan-1,7'-3'H, 5'H-pyrrolo[2',3'-f]-benzimidazol) aus 1'-Acetyl-5',6'-diamino-spiro[cyclopentan-1,3'-indolin] und 4-Methoxy-phenylacetaldehyd | | |
| d) | 5'-Acetyl-2'-[2-(4-methoxy-phenyl)ethenyl]-6',7'-dihydro-spiro(cyclopentan-1,7'-3'H,5'H-pyrrolo[2',3'-f]benzimidazol) aus 1'-Acetyl-5',6'-diamino-spiro[cyclopentan-1,3'-indolin] und 4-Methoxy-zimtaldehyd | | |

Beispiel 22 (Fortsetzung)

| | Bezeichnung | Ausb. % | Schmelzpunkt °C (Lsg.mittel) |
|---|---|---|---|
| e) | 7,7-Dimethyl-6,7-dihydro-2-(3,4-methylendioxy-phenyl)-3H,5H-pyrrolo[2,3-f]benzimidazol-6-on aus 5,6-Diamino-3,3-dimethyl-indolin und Piperonal | 48 | >300 (Ethylacetat) |
| f) | 2'-(3,4-Methylendioxy-phenyl)-6',7'-dihydro-spiro-(cyclopentan-1,7'-3'H,5'H-pyrrolo[2',3'-f]benzimidazol)-6'-on aus 5',6'-Diamino-spiro[cyclopentan-1,3'-indolin]-2'-on und Piperonal | 45 | >300 (Methanol) |
| g) | 2'-(3,4-Ethylendioxy-phenyl)-6',7'-dihydro-spiro(cyclopentan-1,7'-3'H,5'H-pyrrolo-[2',3'-f]benzimidazol)-6'-on aus 5',6'-Diamino-spiro[cyclopentan-1,3'-indolin]-2'-on und 3,4-Ethylendioxy-benzaldehyd | 52 | >300 (Methanol) |

Beispiel 23

In analoger Weise wie in Beispiel 5 beschrieben erhält man:

| | Bezeichnung | Ausb. % | Schmelzpunkt °C (Lsg.mittel) |
|---|---|---|---|
| a) | 7,7-Dimethyl-6,7-dihydro-2-(imidazol-1-yl-methyl)-3H,5H-pyrrolo[2,3-f]benzimidazol-dihydrochlorid aus Verbindung des Beispiels 14 f | 55 | 181-183 (Aceton) |
| b) | 7,7-Dimethyl-6,7-dihydro-2-(4-pyridyl-carbonylamino)-3H-5H-pyrrolo[2,3-f]benzimid-azol-trihydrochlorid aus Verbindung des Beispiels 16 g | 46 | 205-207 (Aceton) |
| c) | 7,7-Dimethyl-2-(2-methoxy-4-methylsulfonyl-phenyl)-6,7-dihydro-3H,5H-pyrrolo[2,3-f]-benzimidazol-dihydrochlorid aus Verbindung des Beispiels 20 | 71 | 267-268 (Ethanol) |
| d) | 7,7-Dimethyl-2-n-hexyl-6,7-dihydro-3H,5H-pyrrolo[2,3-f]-benzimidazol-dihydrochlorid aus Verbindung des Beispiels 22 a | 58 | 181-183 (Aceton) |
| e) | 2'-(4-Methoxy-phenyl)-6',7'-dihydro-spiro(cyclopentan-1,7'-3'H,5'H-pyrrolo[2',3'-f]benzimidazol)-dihydro-chlorid aus Verbindung des Beispiels 22 b | 56 | 268-270 (Aceton) |
| f) | 2'-(4-Methoxy-phenylmethyl)-6',7'-dihydro-spiro(cyclo-pentan-1,7'-3'H,5'H-pyrrolo-[2',3'-f]benzimidazol) aus Verbindung des Beispiels 22 c | 88 | 95-96 (Dichlormethan) |
| g) | 2'-[2-(4-Methoxy-phenyl)-ethenyl]-6',7'-dihydro-spiro-(cyclopentan-1,7'-3'H,5'H-pyrrolo[2',3'-f]benzimid-azol) aus Verbindung des Beispiels 22 d | 54 | 86-88 (Ligroin) |

## Patentansprüche

1. Pyrrolobenzimidazole der Formel I

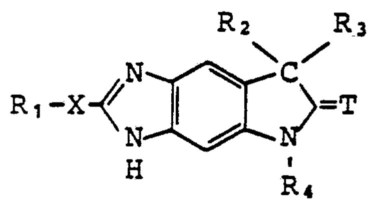

(I)

in welcher

$R_1$ einen Phenylring der allgemeinen Formel II darstellt,

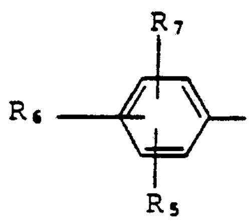

(II),

wobei $R_5$, $R_6$, $R_7$ gleich oder verschieden sein koennen und jeweils Wasserstoff, eine Alkansulfonyloxy-, Trifluormethansulfonyloxy-, Alkansulfonylamino-, Trifluormethansulfonylamino-, N-Alkyl-alkansulfonylamino-, N-Alkyltrifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, eine durch eine Amino-, Alkylamino-, Dialkylamino- oder cyclische Iminogruppe substituierte Sulfonylgruppe, wobei eine Methylengruppe in 4-Stellung durch ein Schwefel- oder Sauerstoffatom ersetzt sein kann, eine Alkylcarbonylamino-, Aminocarbonylamino- oder Alkylaminocarbonylaminogruppe, eine Alkylmercapto-, Alkylsulfinyl- oder Alkylsulfonylgruppe, eine Nitro-, Halogen-, Amino-Hydroxy-, Alkyl-, Alkoxy-, Alkenyloxy-, Alkinyloxy-, Cyanalkyloxy-, Carboxyalkyloxy-, Alkoxycarbonylalkyloxy-, Dialkylamino-,

1-Imidazolyl-, Trifluormethyl- oder Cyangruppe sein koennen, oder $R_1$ einen Naphthylrest bedeutet
oder $R_1$ einen gesaettigten oder ungesaettigten heterocyclischen Fuenfring mit 1-4 Heteroatomen oder einen gesaettigten oder ungesaettigten heterocyclischen Sechsring mit 1-5 Heteroatomen darstellt, wobei die Heteroatome gleich oder verschieden sein koennen und Sauerstoff, Schwefel oder Stickstoff bedeuten und gewuenschtenfalls an einem oder mehreren Stickstoffatomen ein Sauerstoffatom tragen koennen, und die Fuenf- oder Sechsringe gegebenenfalls durch eine oder mehrere Alkyl-, Alkoxy-, Alkylmercapto-, Hydroxy-, Nitro-, Amino-, Halogen- oder Cyangruppen substituiert oder mit einem Phenylring zu einem Bicyclus kondensiert sein koennen,

oder fuer den Fall, daß X eine Bindung darstellt, $R_1$ neben den oben genannten Gruppen auch ein Wasserstoffatom, eine Alkyl-, Cycloalkyl-, Alkenyl-, Cycloalkenyl-, Alkinyl-, Halogenalkyl-, Alkoxyalkyl-, Carboxyalkyl-, Alkoxycarbonylalkyl-, Aminoalkyl-, Hydroxyalkyl-, eine Hydroxy-, Mercapto-, Amino-, Alkylmercapto-, Alkylcarbonylamino-, Formylamino-, Alkylsulfonylamino-, Formylaminoalkyl-, Alkoxycarbonylaminoalkyl- oder Alkylsulfonylaminoalkylgruppe bedeutet,

$R_2$ ein Wasserstoffatom, eine Alkyl-, Alkenyl- oder eine Cycloalkylgruppe bedeutet,

$R_3$ eine Alkyl-, Alkenyl-, oder Hydroxyalkylgruppe bedeutet oder mit $R_2$ zusammen eine Cycloalkylengruppe darstellt oder $R_2$ und $R_3$ zusammen eine Alkyliden- oder Cycloalkylidengruppe bilden,

$R_4$ ein Wasserstoffatom oder einen niederen Alkanoylrest darstellt,

X eine Bindung, eine Alkylen-, die Vinylen-, die Iminogruppe -NH- oder die Carbonylaminogruppe -CONH- bedeutet,

T fuer zwei Wasserstoffatome steht, wobei fuer den Fall, daß X die Iminogruppe -NH- oder die Carbonylaminogruppe

-CONH- bedeutet, oder fuer den Fall, daß $R_1$ einen heterocyclischen Fuenf- oder Sechsring bedeutet, der mit einem Phenylring zu einem Bicyclus kondensiert ist, T auch ein Sauerstoffatom sein kann, deren Tautomere und deren physiologisch vertraegliche Salze anorganischer oder organischer Saeuren.

2. Verbindungen der Formel I gemäß Anspruch 1,

in der

$R_1$ den Phenylrest der allgemeinen Formel II bedeutet, in der $R_5$ ein Wasserstoffatom, die Methansulfonyloxy-, Trifluormethansulfonyloxy-, Methansulfonylamino-, Trifluormethansulfonylamino-, Methylmercapto-, Methylsulfinyl-, Methylsulfonyl-, Hydroxy-, Methyl-, Methoxy-, Propargyloxy-, Trifluormethyl- oder die 1-Imidazolylgruppe,

$R_6$ Wasserstoff, die Methyl-, Methoxy-, Dimethylamino- oder Chlorgruppe,

$R_7$ Wasserstoff oder die Methoxygruppe bedeutet oder

$R_1$ den Pyrrol-, Furan-, Thiophen-, Pyrazol-, Imidazol-, Isothiazol-, Thiazol-, Oxazol-, Triazol-, Tetrazol-, Thiadiazol-, Isoxazol-, Oxadiazol-, Pyridin-, N-Oxy-pyridin-, Pyrazin-, N,N'-Dioxypyrazin, Pyrimidin-, N,N'-Dioxypyrimidin-, Pyridazin-, Oxazin-, Thiazin-, Triazin-, Tetrazin-, Piperidin-, Piperazin-, Morpholin- oder Thiomorpholinrest darstellt sowie deren Methyl-, Ethyl-, Methoxy-, Ethoxy-, Methylmercapto-, Ethylmercapto- und Chlor-substitierten Derivate oder den Indol-, Indazol-, Chinolin- oder Isochinolinrest bedeutet oder einen Naphthylrest darstellt oder
$R_1$ fuer den Fall, daß X eine Bindung darstellt, neben den genannten Gruppen auch ein Wasserstoffatom, die Methyl-, Ethyl-, Propyl-, Butyl-,Pentyl-, Hexyl-, Propenyl-, Cyclopentyl-, Cyclohexyl-, Trifluormethyl-, Hydroxy-, Mercapto-, Methylmercapto-, Amino-, Acetamido- oder Formamidogruppe bedeutet,

$R_2$ und $R_3$ gleich sind und Methylgruppen bedeuten oder $R_2$ und $R_3$ zusammen mit dem Kohlenstoffatom, an das sie gebunden sind, einen Cyclopentanring oder Cyclohexanring bilden,

$R_4$ Wasserstoff, die Formyl-, Acetyl-, Propionyl- oder Butyrylgruppe darstellt und X eine Bindung, die Methylengruppe, die Iminogruppe oder die Carbonylaminogruppe bedeutet.

3. Verfahren zur Herstellung von Pyrrolobenzimidazole der Formel I

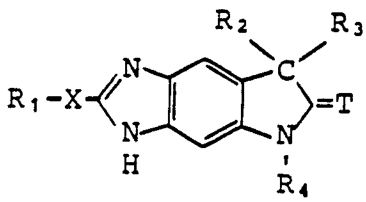

(I)

in welcher

$R_1$ einen Phenylring der allgemeinen Formel II darstellt,

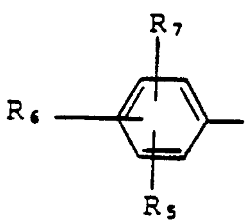

(II),

wobei $R_5$, $R_6$, $R_7$ gleich oder verschieden sein koennen und jeweils Wasserstoff, eine Alkansulfonyloxy-, Trifluormethansulfonyloxy-, Alkansulfonylamino-, Trifluormethansulfonylamino-, N-Alkyl-alkansulfonylamino-, N-Alkyltrifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, eine durch eine Amino-, Alkylamino-, Dialkylamino- oder cyclische Iminogruppe substituierte Sulfonylgruppe, wobei eine Methylengruppe in 4-Stellung durch ein Schwefel- oder Sauerstoffatom ersetzt sein kann, eine Alkylcarbonylamino-, Aminocarbonylamino- oder Alkylaminocarbonylaminogruppe, eine Alkylmercapto-, Alkylsulfinyl- oder Alkylsulfonylgruppe, eine Nitro-, Halogen-, Amino-, Hydroxy-, Alkyl-, Alkoxy-, Alkenyloxy-, Alkinyloxy-, Cyanalkyloxy-, Carboxyalkyloxy-, Alkoxycarbonylalkyloxy-, Dialkylamino-,

1-Imidazolyl-, Trifluormethyl- oder Cyangruppe sein koennen, oder $R_1$ einen Naphthylrest bedeutet oder $R_1$ einen gesaettigten oder ungesaettigten heterocyclischen Fuenfring mit 1-4 Heteroatomen oder einen gesaettigten oder ungesaettigten heterocyclischen Sechsring mit 1-5 Heteroatomen darstellt, wobei die Heteroatome gleich oder verschieden sein koennen und Sauerstoff, Schwefel oder Stickstoff bedeuten und gewuenschtenfalls an einem oder mehreren Stickstoffatomen ein Sauerstoffatom tragen koennen, und die Fuenf- oder Sechsringe gegebenenfalls durch eine oder mehrere Alkyl-, Alkoxy-, Alkylmercapto-, Hydroxy-, Nitro-, Amino-, Halogen- oder Cyangruppen substituiert oder mit einem Phenylring zu einem Bicyclus kondensiert sein koennen,

oder fuer den Fall, daß X eine Bindung darstellt, $R_1$ neben den oben genannten Gruppen auch ein Wasserstoffatom, eine Alkyl-, Cycloalkyl-, Alkenyl-, Cycloalkenyl-, Alkinyl-, Halogenalkyl-, Alkoxyalkyl-, Carboxyalkyl-, Alkoxycarbonylalkyl-, Aminoalkyl-, Hydroxyalkyl-, eine Hydroxy-, Mercapto-, Amino-, Alkylmercapto-, Alkylcarbonylamino-, Formylamino-, Alkylsulfonylamino-, Formylaminoalkyl-, Alkoxycarbonylaminoalkyl- oder Alkylsulfonylaminoalkylgruppe bedeutet,

$R_2$ ein Wasserstoffatom, eine Alkyl-, Alkenyl- oder eine Cycloalkylgruppe bedeutet,

$R_3$ eine Alkyl-, Alkenyl-, oder Hydroxyalkylgruppe bedeutet oder mit $R_2$ zusammen eine Cycloalkylengruppe darstellt oder $R_2$ und $R_3$ zusammen eine Alkyliden- oder Cycloalkylidengruppe bilden,

$R_4$ ein Wasserstoffatom oder einen niederen Alkanoylrest darstellt,

X eine Bindung, eine Alkylen-, die Vinylen-, die Iminogruppe -NH- oder die Carbonylaminogruppe -CONH- bedeutet,

T fuer zwei Wasserstoffatome steht, wobei fuer den Fall, daß X die Iminogruppe -NH- oder die Carbonylaminogruppe -CONH- bedeutet, oder fuer den Fall, daß $R_1$ einen heterocyclischen Fuenf- oder Sechsring bedeutet, der mit einem Phenylring zu einem Bicyclus kondensiert ist, T auch ein Sauerstoffatom sein kann,

deren Tautomere und deren physiologisch vertraegliche Salze anorganischer oder organischer Saeuren, dadurch gekennzeichnet, daß man

a) eine Verbindung der allgemeinen Formel III

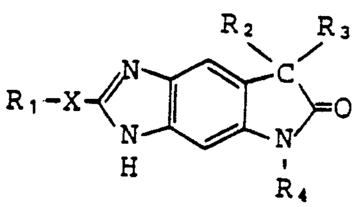

(III),

in welcher $R_1$, $R_2$, $R_3$, $R_4$ und X die obengenannte Bedeutung haben, reduziert oder

b) eine Verbindung der allgemeinen Formel IV a oder IV b

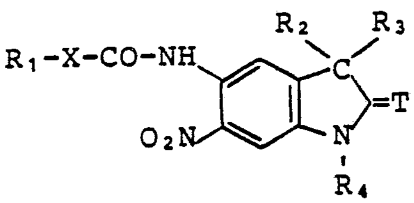 (IV a) 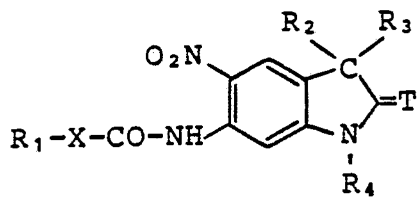 (IV b),

in welcher $R_1$, $R_2$, $R_3$, $R_4$, T und X die obengenannte Bedeutung haben, reduziert und cyclisiert oder

c) eine Verbindung der allgemeinen Formel V

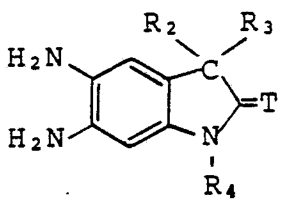 (V),

in welcher T, $R_2$, $R_3$ und $R_4$ die obengenannte Bedeutung haben, mit einer Verbindung der allgemeinen Formel VI

$$R_1'-X-\underset{\overset{\shortparallel}{W}}{C}-Y \qquad (VI),$$

in welcher X die oben angegebene Bedeutung besitzt, Y ein Wasserstoffatom, die Hydroxygruppe oder eine leicht abspaltbare Gruppe bedeutet, W fuer ein Sauerstoffatom steht oder gemeinsam mit Y ein Stickstoffatom darstellt und $R_1'$ die oben fuer $R_1$ angegebene Bedeutung besitzt mit der Ausnahme, daß fuer den Fall, daß X eine Bindung darstellt,

$R_1$' nicht die Amino-, Hydroxy- oder Mercaptogruppe bedeutet, oder

mit Verbindungen umsetzt, die Carbonyl-, Thiocarbonyl- oder Iminogruppen uebertragen wie beispielsweise Phosgen, Thiophosgen, 1,1'-Carbonyldiimidazol, Chlorameisensaeureester, Kohlensaeureester, Harnstoff oder Bromcyan,

und anschließend gewuenschtenfalls

die erhaltene Verbindung der allgemeinen Formel I oder deren Tautomer in eine andere Verbindung der allgemeinen Formel I umwandelt und/oder die erhaltene Verbindung der allgemeinen Formel I in ein physiologisch vertraegliches Salz einer organischen oder anorganischen Saeure ueberfuehrt.

4. Verfahren zur Herstellung von Verbindungen der Formel I gemäß Anspruch 3,

in der

$R_1$ den Phenylrest der allgemeinen Formel II bedeutet, in der $R_5$ ein Wasserstoffatom, die Methansulfonyloxy-, Trifluormethansulfonyloxy-, Methansulfonylamino-, Trifluormethansulfonylamino-, Methylmercapto-, Methylsulfinyl-, Methylsulfonyl-, Hydroxy-, Methyl-, Methoxy-, Propargyloxy-, Trifluormethyl- oder die 1-Imidazolylgruppe,

$R_6$ Wasserstoff, die Methyl-, Methoxy-, Dimethylamino- oder Chlorgruppe,

$R_7$ Wasserstoff oder die Methoxygruppe bedeutet oder
$R_1$ den Pyrrol-, Furan-, Thiophen-, Pyrazol-, Imidazol-, Isothiazol-, Thiazol-, Oxazol-, Triazol-, Tetrazol-, Thiadiazol-, Isoxazol-, Oxadiazol-, Pyridin-, N-Oxy-pyridin-, Pyrazin-, N,N'-Dioxypyrazin, Pyrimidin-, N,N'-Dioxypyrimidin-, Pyridazin-, Oxazin-, Thiazin-, Triazin-, Tetrazin-, Piperidin-, Piperazin-, Morpholin- oder Thiomorpholinrest darstellt sowie deren Methyl-, Ethyl-, Methoxy-, Ethoxy-, Methylmercapto-,

Ethylmercapto- und Chlor-substitierten Derivate oder den Indol-, Indazol-, Chinolin- oder Isochinolinrest bedeutet oder einen Naphthylrest darstellt oder

$R_1$ fuer den Fall, daß X eine Bindung darstellt, neben den genannten Gruppen auch ein Wasserstoffatom, die Methyl-, Ethyl-, Propyl-, Butyl-,Pentyl-, Hexyl-, Propenyl-, Cyclopentyl-, Cyclohexyl-, Trifluormethyl-, Hydroxy-, Mercapto-, Methylmercapto-, Amino-, Acetamido- oder Formamidogruppe bedeutet,

$R_2$ und $R_3$ gleich sind und Methylgruppen bedeuten oder $R_2$ und $R_3$ zusammen mit dem Kohlenstoffatom, an das sie gebunden sind, einen Cyclopentanring oder Cyclohexanring bilden,

$R_4$ Wasserstoff, die Formyl-, Acetyl-, Propionyl- oder Butyrylgruppe darstellt und X eine Bindung, die Methylengruppe, die Iminogruppe oder die Carbonylaminogruppe bedeutet.

5. Arzneimittel enthaltend eine Verbindung gemäß Anspruch 1 oder 2, neben üblichen Träger- und Hilfsstoffen.

6. Verwendung von Verbindungen gemäß Anspruch 1 oder 2 zur Behandlung von Herz- und Kreislauferkrankungen.